# EUROPEAN PATENT APPLICATION

(11) **EP 2 898 774 A1**
(43) Date of publication of application: **29.07.2015**
(21) Application number: 14000292.4
(22) Date of filing: 28.01.2014
(51) Int. Cl.: A01N 25/28, A61K 8/11, C11D 17/00, C11D 1/835, C11D 3/22, C11D 1/72

(54) **Composition for the treatment of textile materials**

(71) Applicant: Archroma IP GmbH, 4153 Reinach (CH); Lipotec S.A., 08850 Gavà Barcelona (ES)
(72) Inventor: Steffanut, Pascal, CH-4153 Reinach (CH); Viladot-Petit, Joseph-Lluis, 08850 Gava, Barcelona (ES)
(74) Representative: Ricker, Mathias

(57) **Abstract**

Composition comprising:
(a) at least one cationic microcapsule having a shell and a core;
(b) water;
(c) at least one non-ionic oxygen-containing compound comprising at least one alkylene group;
(d) at least one quaternary ammonium compound.

The composition may be used for the treatment of textile materials. Substrates containing this composition may be used for the treatment and/or care of the skin, hair and/or scalp.

## Description

### FIELD OF THE INVENTION

This invention refers to aqueous cationic microcapsule dispersions having improved long term stability, to a process of making said dispersions, and to the use of said dispersions in textile, cosmetic and therapeutic applications.

### BACKGROUND OF THE INVENTION

Techniques for encapsulation of cosmetic and/or pharmaceutical active ingredients in microcapsules are known. Encapsulation techniques consist of the coating of active ingredients which are useful in different fields such as cosmetics, pharmacy or food in the form of different natured polymers to obtain particles of a size comprised between 1 µm and 1 mm. [E. G. JalonDe, M. J. Blanco-Prieto, P. Ygartua, and S. Santoyo. Eur. J. Pharm. Biopharm. 56:183-187 (2003*);* M. A. Augustin and Y. Hemar. Chem. Soc. Rev., 2009, 38, 902 - 912*;* Sofia N. Rodrigues, Isabel Fernandes, Isabel M. Martins, Vera G. Mata, Filomena Barreiro, and Alirio E. Rodrigues. Ind. Eng. Chem. Res.2008, 47 (12), 4142-4147*;* Stephan Drusch and Saverio Mannino. Trends in Food Science & Technology 2009, 20, 237-244*].*

A microcapsule has a relatively simple morphological structure, and is comprised by two clearly differentiated elements, a nucleus which contains one or several active ingredients and a polymeric coating or surface which surrounds the nucleus and thus protects the active ingredients from the outside [W. Sliwka. Angew. Chem. Internat. Edit. 14, 539 (1975*)*]*.* In the following, the coating or surface is also termed as *"shell'* and the nucleus is termed as *"core".*

One of the microencapsulation applications which has stirred up great interest in recent years is the treatment of fibers and textile materials in order to improve their aspect, shine, color, smell and elasticity or to provide new the fiber or textile material with functionalities such as the treatment and/or care of the skin, scalp and/or hair. Microencapsulation is a solution often used to bind the encapsulated active ingredient to the fiber or textile material since it ena-enables to prolong the permanence of the active ingredient in the fiber or textile material, and which is slowly released onto the skin, scalp and/or hair by degradation of the microcapsules by pressure, heat, friction, osmosis or body moisture. Thus a continual release of active ingredients onto the skin, scalp and/or hair is achieved through the textile materials.

Different types of binding or linking of microcapsules to fibers or textile materials are known, for example the binding of microcapsules to fibers or textile materials through covalent bonds [W.Chao-Xia and C. Shui-Lin, Coloration Technology 2004, 120, 14-18*].* In a typical embodiment, the microcapsule coating is formed by cyclodextrins, and preferably, the encapsulated active ingredients are perfumes or odor-trapping agents. The fact that the microcapsules are bound or linked covalently fixes them on the fibers or textile materials, and the microcapsules of the active ingredient to be encapsulated can be reloaded. The principal problem presented by this type of binding is that the creation of covalent bonds entails a modification to the chemical structure of the fiber, which can lead to degradation and breakage of the textile material.

Another type of binding or linking of microcapsules to fibers or textile materials is the manufacture of fibers in solutions, wherein the microcapsules containing the active ingredients which are to be fixed to the fibers are already present [Qi, Ping Hu, Jun Xu, and Wang Biomacromolecules, 2006, 7 (8), 2327-2330]. This type is limited solely to active ingredients which should not pass through to the skin, such as insect repellents, and which, therefore, excludes any type of cosmetically and/or pharmaceutically used active ingredient.

Another type of binding or linking of microcapsules to fibers or textile materials is through ionic bonds between the microcapsule and the fibers [P. Monllor, L. Sánchez, F. Cases and M. A. Bonet, Textile Research Journal 2009, 79, 365-380]. The binding is caused by electrostatic interaction between the negative charges of the fibers of the textile materials and the microcapsules which have previously been cationized. The principal advantages of this type of binding or link over the previous types are that it is not limited to one type of active ingredient and that this type of binding to the fibers or textile materials is not detrimental to the mechanical properties of the textile material as in the case of covalent binding. In addition, and also unlike the previous types of binding of microcapsules containing active ingredients to textile materials, there is the possibility of the final consumer reloading the textile material with microencapsulated active ingredients himself.

WO 01/62376 A1 relates to microcapsules and/or nanocapsules, whose surfaces have positive charges, exhibit a specific affinity to substrates such as textiles, hair or skin so that, even after having been treated with water, at least up to a certain portion thereof remains on these substrates. The inventive capsules can be diversely used, in particular, in washing and cleaning agents as well as in cosmetics.

### OBJECT OF THE INVENTION

However, the main problems that arise with cationically charged microcapsules containing active ingredients are the low physical stability of the dispersion of these cationically charged microcapsules upon dilution and the wash-fastness both of the cationically charged microcapsules and/or the active ingredients contained in the cationically charged microcapsules when they come into contact with the surfactants of the washing agents of textile materials. These problems are both deeply related to the interaction of the microcapsule surface with the external surrounding upon textile application or dispersion preparation processes. Therefore, there is a need for resolving the aforementioned problems, respectively to provide dispersions having improved properties.

### SUMMARY OF THE INVENTION

The present invention overcomes the problem of the low physical stability of the dispersions of cationically charged microcapsules containing active agents (active ingredients), respectively the present invention at least provides improved dispersions.

Accordingly, the invention relates to a dispersion containing cationically charged microcapsules and a combination of specific dispersing agents and surfactants. Additionally, the ingredients of the composition of the invention are in the list of the chemical ingredients for the cosmetic industry approved by the European Community.

Specifically, according to a ***first aspect,*** the invention relates to a composition comprising:
(a) at least one cationic microcapsule having a shell and a core, wherein the microcapsule comprises at least one active agent;
(b) water;
(c) at least one non-ionic oxygen-containing compound comprising at least one alkylene group;
(d) at least one quaternary ammonium compound.

In one embodiment, the the shell of the microcapsule consists or comprises at least a cationic polymer and/or is partially covered with at least a cationic compound.

In one embodiment, the active agent is selected from the group consisting of cosmetic and/or pharmaceutical active agents and insect repellants.

In one embodiment, the said at least one compound (c) is a polyalkylene glycol ether.

In one embodiment, said polyalkylene glycol ether is a reaction product of a C₆₋₂₆ alcohol with ethylene oxide or propylene oxide.

In one embodiment, said at least one compound (c) is a polyalkylene glycol ether of formula (i)

R₁-O-(CH₂-CHR₂)ₙ-OH (i)

wherein R₁ is a branched or unbranched alkyl or alkenyl group having from 6 to 26 carbon atoms; and R₂ is H or CH₃; or said ether of formula (i) comprises moieties in which is R₂ is H and moieties in which R₂ is CH₃; and wherein n is a integer in the range of from 1 to 300.

In one embodiment, said at least one compound (c) of formula (i) is a polyalkylene glycol ether of formula (i.1)

CH₃-(CH₂)ₘ-(O-CH₂-CH₂)ₙ-OH (i.1)

or of formula (i.2)

CH₃-(CH₂)ₘ-(O-CH₂-CHCH₃)ₙ-OH (i.2)

wherein m is an integer in the range of from 5 to 25 and n is an integer in the range of from 1 to 300, preferably wherein m is in the range of from 11 to 23 and n is in the range of from 1 to 200 or 2 to 150 or 2 to 100.

In one embodiment, said at least one compound (c) is selected from the group consisting of a polyoxyethylene ether of laurylalcohol, cetyl alcohol, cetyl stearyl alcohol, stearyl alcohol, oleyl alcohol, and tridecyl alcohol, and a mixture of two or more thereof.

In one embodiment, said at least one compound (c) is a polyoxyethylene stearyl ether selected from the group consisting of polyoxyethylene (2) stearyl ether (Steareth-2), polyoxyethylene (3) stearyl ether (Steareth-3), polyoxyethylene (4) stearyl ether (Steareth-4), polyoxyethylene (5) stearyl ether (Steareth-5), polyoxyethylene (6) stearyl ether (Steareth-6), polyoxyethylene (7) stearyl ether (Steareth-7), polyoxyethylene (8) stearyl ether

(Steareth-8), polyoxyethylene (10) stearyl ether (Steareth-10), polyoxyethylene (15) stearyl ether (Steareth-15), polyoxyethylene (16) stearyl ether (Steareth-16), polyoxyethylene (20) stearyl ether (Steareth-20), polyoxyethylene (21) stearyl ether (Steareth-21), polyoxyethylene (25) stearyl ether (Steareth-25), polyoxyethylene (30) stearyl ether (Steareth-30), polyoxyethylene (40) stearyl ether (Steareth-40), polyoxyethylene (50) stearyl ether (Steareth-50), polyoxyethylene (100) stearyl ether (Steareth-100) and polyoxyethylene (200) stearyl ether (Steareth-200), and a mixture of two or more thereof.

In one embodiment, the concentration of said at least one compound (c) is in the range of from 1 to 10 % by weight or 2 to 5 % by weight or 3 to 4 % by weight based on the total weight of the composition.

In one embodiment, said at least one compound (d) is selected from the group consisting of a compound of formula (ii)

N(R₁, R₂, R₃, R₄)⁺ X⁻ (ii)

wherein R₁, R₂, R₃, R₄ independently are acyclic C₁₋₂₄ residues; or wherein
R₁ is an acyclic C₁₂₋₂₄ residue, and R₂ to R₄ independently are C₁₋₄alkyl residues or C₁₋₄ hydroxyalkyl residues; and
X⁻ is selected from halogenide, methosulfate, metophosphate, phosphate, sulfate, hydroxide, carbonate and hydrogen carbonate, preferably halogenide, methosulfate, metophosphate and phosphate;
and a compound of formula (iii)

   quaternary ammonium compound comprising a hydroxypropyltrimonium⁺ X⁻ moiety (iii)

   wherein X- is selected from halogenide, methosulfate, metophosphate, phosphate, sulfate, hydroxide, carbonate and hydrogen carbonate, preferably halogenide, methosulfate, metophosphate and phosphate;
   and a mixture of compounds (ii) and (iii).

In one embodiment, said compound of formula (iii) is a quaternary ammonium compound comprising a hydroxypropyltrimonium⁺X⁻ moiety and a carbohydrate moiety.

In one embodiment, said carbohydrate is a sugar or a starch.

In one embodiment, the composition comprises at least one compound of formula (iii) or comprises at least one compound of formula (ii) and at least one compound of formula (iii).

In one embodiment, said at least one compound (d) of formula (ii) is selected from the group consisting of lauryl trimethyl ammonium X⁻, cetyl trimethyl ammonium X⁻, cetyl stearyl trimethyl ammonium X⁻, stearyl trimethyl ammonium X⁻, docosyl trimethyl ammonium (behentrimonium) X⁻, oleyl trimethyl ammonium X⁻, lauryl dimethyl hydroxyethyl ammonium X⁻, cetyl dimethyl hydroxyethyl ammonium X⁻, cetylstearyl dimethyl hydroxyethyl ammonium X⁻, stearyl dimethyl hydroxyethyl ammonium X⁻, docosyl dimethyl hydroxyethyl ammonium (behentrimonium) X⁻, oleyl dimethyl hydroxyethyl ammonium X⁻,
and/or
wherein said at least one compound (d) of formula (iii) is selected from starch hydroxypropyltrimonium X⁻, cassia hydroxylpropytrimonium X⁻ and guar hydroxypropyltrimonium X⁻;
or a mixture of two or more of compounds of formula (ii) and (iii).

In one embodiment, the concentration of the compound of formula (ii) is from 1 to 3 % by weight or from 1.5 to 2 % by weight based on the total amount of the composition and/or wherein the concentration of the compound of formula (iii) is in the range of from 0.1 to 2 % by weight or from 0.25 to 0.5 % by weight based on the total amount of the composition.

In one embodiment, said at least one compound (c) is a polyoxyethylene stearyl ether of formula (i), preferably selected from the group consisting of Steareth-10, Steareth-15 or Steareth-20, and said at least one compound (d) of formula (ii) is behentrimonium chloride or lauryl dimethyl hydroxyethyl ammonium chloride in combination with cassia hydroxypropyltrimonium chloride of formula (iii).

In one embodiment said at least one compound (c) is a polyoxyethylene stearyl ether of formula (i) at a concentration from 2 % to 4.0 % by weight, preferably selected from the group consisting of Steareth-10, Steareth-15 or Steareth-20, and said at least one compound (d) of formula (ii) is behentrimonium chloride or lauryl dimethyl hydroxyethyl ammonium chloride at a concentration of from 1.0 to 3.0 % by weight in combination with cassia hydroxypropyltrimonium chloride of formula (iii) at a concentration of from 0.1% to 1.0 % by weight.

According to a ***second aspect**,* the invention relates to a process for the manufacture of the composition as defined in the ***first aspect,*** comprising:
mixing at least one cationic microcapsule having a shell and a core (a), wherein the microcapsule comprises at least one active agent, with water (b) and compounds (c) and (d).

In one embodiment, the temperature is in the range of from 15 °C to reflux temperature.

According to a ***third aspect**,* the invention relates to a substrate comprising the composition as defined in the ***first** aspect.*

In one embodiment, the substrate is selected from a natural or synthetic or semisynthetic material and blends of two or more thereof.

In one embodiment, the material contains natural, modified or regenerated cellulose, natural or synthetic polyamide or fully synthetic polyamides, polyester, polyurethane or polyacrylonitrile, and blends of two or more thereof.

In one embodiment, the substrate comprising the composition as defined in the ***first aspect*** has been dried, preferably at a temperature of from 30 to 190°C.

According to a ***fourth aspect**,* the invention relates to a fabric softener comprising the composition as defined in the ***first aspect.***

According to a ***fifth aspect,*** the invention relates to the use of the substrate as defined in the ***third aspect*** for the cosmetic, non-therapeutic treatment and/or care of the skin, hair and/or scalp.

In one embodiment, the cosmetic, non-therapeutic treatment and/or care of the skin, hair and/or scalp is selected from the group formed by the treatment and/or prevention of skin aging, treatment and/or prevention of cellulite, moisturizing of the skin, firming of the skin, and/or whitening of the skin.

According to a *sixth aspect,* the invention relates to a substrate as defined in the *third aspect* for use as a medicament.

In one embodiment, the substrate is for use in the treatment of the skin and/or scalp and/or in the prevention of skin diseases.

In one embodiment, the the treatment is the healing of the skin and/or scalp.

### DESCRIPTION OF THE INVENTION

In a ***first aspect,*** the present invention relates to a composition comprising:
(a) at least one cationic microcapsule having a core and a shell, wherein the microcapsule comprises at least one active agent;
(b) water;
(c) at least one non-ionic oxygen-containing compound comprising at least one alkylene group;
(d) at least one quaternary ammonium compound.

As used herein, the term *"comprising"* which is synonymous with *"including," "containing"* or *"characterized by"* is inclusive or open-ended and does not exclude additional, un-recited elements or method steps. However, in each recitation of *"comprising"* herein, it is intended that the term also encompasses, as alternative embodiments, the phrases *"consisting essentially of"* and *"consisting of"* where *"consisting of"* excludes any element or step not specified and *"consisting essentially of"* permits the inclusion of additional un-recited elements or steps that do not materially affect the essential or basic and novel characteristics of the composition or method under consideration.

### Component/compound (a)

The term *"microcapsule having a core and a shelf'* encompasses the respective meaning as referred to in the Background Section.

The term *"cationic microcapsule"* is synonymously used to the term *"cationically charged microcapsule".*

The shell of the microcapsule consists or comprises at least a cationic polymer and/or is partially covered with at least a cationic compound, preferably wherein said cationic compound is at least one quaternary ammonium compound (d).

Preferably, the composition according to the invention comprises as component/compound (a) a multitude of such cationically charged microcapsules.

### Shell of the cationic microcapsule

In one embodiment, the shell of the cationic microcapsule is a polymeric shell comprising proteins and polysaccharides, for example and not restricted to, from the group formed by gelatin, albumin, β-lactoglobulin, whey protein, pea protein, potato protein, broad bean protein, wheat protein, bovine serum albumin, poly-L-lysine, soy protein, caseinates, casein, soy glycine, sodium alginate, wheat starch, corn starch, methyl cellulose, ethyl cellulose, hydroxypropyl methyl cellulose (HPMC), cellulose nitrate, carboxymethyl cellulose, gum arabic, xanthan gum, mesquite gum, guar gum, carrageenans, tragacanth gum, arabinogalactans, galactomannans, sodium hexametaphosphate, exopolysaccharide B40, carboxymethyl sodium, pectin, methoxyl pectin, agar, dextran, chitosan, cellulose acetate butyrate, cellulose acetate phthalate, acrylic derivatives and polyesters such as poly-ε-caprolactone, zein, hydroxypropyl methylcellulose phthalate, hydroxypropyl methylcellulose acetate succinate, polyvinyl acetate phthalate, poly(p-dioxanone), poly(δ-valerolactone), poly(β-hydroxybutyrate), poly(β-hydroxybutyrate) and β-hydroxyvalerate copolymers, poly(β-hydroxypropionate), methylacrylic acid copolymers (Eudragit^{®} L and S), dimethylaminoethyl methacrylate copolymers (Eudragit^{®} E), trimethylammonium ethyl methacrylate copolymers (Eudragit^{®} RL and RS), polymers and copolymers of lactic and glycolic acid, polymers and copolymers of lactic and glycolic acid and polyethylene glycol and mixtures thereof. In one embodiment the polymeric shell of the cationically charged microcapsules is composed of gelatin and/or carboxymethyl cellulose.

In one embodiment, the cationic polymer linked to the polymeric shell of the cationic microcapsules is selected, for example and not restricted to, from the group formed by cationic derivatives of cellulose, such as quaternized hydroxyethyl cellulose, which can be acquired under the name Polymer JR 400™ by Amerchol; cationic starches; diallyl ammonium and acrylamide salt copolymers; quaternized vinylpyrrolidone/vinylimidazole polymers such as Luviquat™ (BASF); condensation products of polyglycols and amines; polyquaternium polymers and copolymers, polyquaternium-6, polyquaternium-7, polyquaternium-16, polyquaternium-10; polyquaternium-4 copolymers (Merquat™ polymers by Lubrizol); dicocoylethylhydroxyethylammonium, grafting copolymers with a cellulose skeleton and quaternary ammonium groups; quaternized collagen polypeptides such as hydroxypropyllauryldimonium hydrolyzed collagen (Lamequat™ by Grünau); quaternized wheat polypeptides; polyethylenimine; cationic silicone polymers such as amidomethicone or quaternium-22 silicone; adipic acid and dimethyla-dimethylamino hydroxypropyl diethylenetriamine copolymers (Cartaretine™ by Clariant); acrylic acid copolymers with dimethyldiallylammonium chloride; cationic chitin derivatives such as chitosan and its derivatives; condensation products of cationic dihalogen alkylene such as dibromobutane with bisdialkylamines; bis-dimethylamino-1,3-propane; derivatives of cationic guar gum such as guar-hydroxypropyltrimonium, Jaguar™ CBS, Jaguar™ C-17, Jaguar™ C-16 by Celanese; quaternary ammonium salt polymers such as Mirapol™ A-15, Mirapol™ AD-1, Mirapol™ AZ-1 by Miranol; quaternized polysaccharide polymers of natural derivatives such as azarose; cationic proteins selected from gelatin, gum arabic; cationic polymers from the group formed by polyamides, polycyanoacrylates, polylactides, polyglycolides, polyaniline, polypyrrole, polyvinylpyrrolidone, amino silicone polymers and copolymers, polystyrene, polyvinyl alcohol, polystyrene and maleic acid anhydride copolymers, methyl vinyl ether, epoxy resins and styrene and methyl methacrylate copolymers; cationic polyacrylates and polymethacrylates such as Eudragit™ RL 30 D by Röhm; polyamine derivatives optionally substituted by derivative polyethylene glycol members; polyamino acids under pH conditions wherein they are cationic; polyethyleneimine; quaternized derivatives of polyvinylpyrrolidone (PVP) and hydrophilic urethane polymers, as well as any mixture of the aforementioned cationic groups. In another embodiment the cationic polymer is selected, for example and not restricted to, from the group formed by starch hydroxypropyltrimonium chloride (Sensomer™ CI-50 by Lubrizol), dicocoylethyl hydroxyethylmonium methosulfate, cetrimonium chloride, distearoylethyl hydroxyethylmonium methosulfate, dipalmitoylethyl hydroxyethylmonium methosulfate, babassuamidopropalkonium chloride, trimonium acetamide propyl chloride, cetrimonium tosylate, oleamidopropyl hydroxysultaine, quaternium-22, quaternium-91, dilaureth-4 dimonium chloride, distearyldimonium chloride, PEG-5 hydrogenated tallow amine, trimethylsilyl propyl soy dimonium chloride, minoxidil, capryloyl glycine, hydroxypropyltrimonium hydrolyzed corn starch, cysteine chlorhydrate, biotin, carnitine, ceramide 2, ceramide 1, ceramide 6, alanine, cocamidoethyl betaine, cystine, cocodimonium hydroxypropyl hydrolyzed rice protein, DEA-isostearate, DEA-lauraminopropionate, disodium lauriminodiacetate. In another embodi-embodiment, the cationic polymer is starch hydroxypropyltrimonium chloride (Sensomer™ CI-50 by Lubrizol).

### Core of the cationic microcapsule

The at least one microcapsule contains one or several active ingredients, also termed as *"active agents".* Active agents may be selected from the group consisting of cosmetic and/or pharmaceutical active agents, preferably dermopharmaceutical active ingredients, and/or excipients, and insect repellents.

The term *"cosmetic or dermopharmaceutical active agent"* encompasses a non-toxic but sufficient quantity of the active agent to provide the desired effect. In one embodiment, the cosmetic or dermopharmaceutic concentrations of the active agents is between 0.0000000001 % and 20 % by weight, between 0.00000001 % and 10 % by weight, between 0.000001 % and 5 % by weight, between 0.0001 % and 5 % by weight with regard to the total weight of the composition of the invention.

In one embodiment, the one or more cosmetic and/or dermopharmaceutical active agents and/or excipients are selected, for example and not restricted to, from the group formed by lipolytic agents or agents stimulating lipolysis, venotonic agents, agents modulating PGC-1α expression, agents inhibiting the activity of PPARγ, agents which reduce the triglyceride content of adipocytes, anti-cellulite agents, agents delaying adipocyte differentiation, agents which diminish the sebum production, agents that reduces the amount of nocturnin, agents inhibiting the nocturnin expression, agents stimulating the synthesis of dermal or epidermal macromolecules and/or capable of inhibiting or preventing their degradation, collagen synthesis-stimulating agents, elastin synthesis-stimulating agents, decorin synthesis-stimulating agents, laminin synthesis-stimulating agents, defensin synthesis-stimulating agents, chaperone synthesis-stimulating agents, cAMP synthesis-stimulating agents, agents that modulate AQP-3, agents that modulate aquaporin synthesis, proteins from the aquaporin family, hyaluronic acid synthesis-stimulating agents, glycosaminoglycan synthesis-stimulating agents, fibronectin synthesis-stimulating agents, sirtuin synthesis-stimulating agents, heat shock proteins, heat shock protein synthesis-stimulating agents, agents which inhibit neuronal exocytosis, anticholinergic agents, agents which inhibit muscular contraction, anti-aging agents, anti-wrinkle agents, antiperspirant agents, anti-inflammatory agents and/or analgesics, anti-itching agents, calming agents, anesthetic agents, inhibitors of acetylcholine-receptor aggregation, agents that inhibit acetylcholinesterase, skin relaxant agents, melanin synthesis stimulating or inhibiting agents, whitening or depigmenting agents, propigmenting agents, self-tanning agents, NO-synthase inhibiting agents, 5α-reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, skin conditioners, humectants, substances that retain moisture, alpha hydroxy acids, beta hydroxy acids, moisturizers, epidermal hydrolytic enzymes, vitamins, amino acids, proteins, pigments or colorants, dyes, biopolymers, gelling polymers, thickeners, surfactants, softening agents, emulsifiers, binding agents, preservatives, agents able to reduce or treat the bags under the eyes, exfoliating agents, keratolytic agents, desquamating agents, antimicrobial agents, antifungal agents, fungistatic agents, bactericidal agents, bacteriostatic agents, agents stimulating the synthesis of lipids and components of the stratum corneum, ceramides, fatty acids, agents that inhibit collagen degradation, agents that inhibit matrix metalloproteinase, agents that inhibit elastin degradation, agents that inhibit serine proteases such as kallikreins, leukocyte elastase or cathepsin G, agents stimulating fibroblast proliferation, agents stimulating keratinocyte proliferation, agents stimulating melanocyte proliferation, agents stimulating keratinocyte differentiation, antihyperkeratosis agents, comedolytic agents, anti-psoriasis agents, DNA repair agents, DNA protecting agents, stem cell protecting agents, stabilizers, agents for the treatment and/or care of sensitive skin, firming agents, anti-stretch mark agents, binding agents, agents which inhibit the activity of PAR-2, agents stimulating healing, coadjuvant healing agents, agents stimulating reepithelialization, coadjuvant reepithelialization agents, cytokine growth factors, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, hair loss retardant agents, preservatives, perfumes, cosmetic and/or absorbent and/or body odor masking deodorants, agents inhibiting sweat-degrading enzymes, chelating agents, plant extracts, essential oils, marine extracts, agents obtained from a biotechnological process, mineral salts, cell extracts, sunscreens and organic or mineral photoprotective agents active against ultraviolet A and/or B rays and/or infrared A rays, or mixtures thereof, provided that they are physically and chemically compatible with the rest of components in the composition. Likewise, the nature of these additional ingredients should not unacceptably alter the benefits of the extract of this invention. The nature of these additional ingredients can be synthetic or natural, such as plant extracts, or come from a biotechnological procedure, or from a combination of a synthetic procedure and a biotechnological procedure. Additional examples can be found in CTFA International Cosmetic Ingredient Dictionary & Handbook, 12th Edition (2008*).* In the context of this invention, biotechnological procedure is understood to be any procedure to produce the active ingredient, or part of it, in an organism, or in part of it. The amount of the cosmetic and/or dermopharmaceutical active ingredients and/or excipients in the composition of the invention is a cosmetically or dermopharmaceutically effective quantity.

In the following, some of the active agents are disclosed in more detail.

### Specific active agents

In one embodiment, the agent that reduces the triglyceride content of adipocytes, agent that delays adipocyte differentiation, anti-cellulite agent, lipolytic agent, venotonic agent, agent inhibiting PGC-1α expression or agent inhibiting the activity of PPARγ is selected, for example and not restricted to extracts or hydrolyzed extracts of *Agave americana, Agave sisalana, Alchemilla vulgaris, Anemarrhena apshodeloides, Angelica sinensis, Armeniacea sp., Arnica montana L, Asparagus adscende, Asparagus cochichinensis, Asparagus filicinus, Asparagus meioclados, Asparagus munitus, Asparagus myriancanthus, Asparagus officinalis, Asparagus racemosus, Asparagus taliensis, Asparagus trichoclados, Atractylodis platicodon,* bamboo, *Betula alba, Bupleurum chinensis, Calendula officinalis,* cangzhu, *Cecropia obtusifolia, Celosia cristata, Centella asiatica, Chenopodium quinoa, Chrysanthellum indicum, Cimifuga racemosa, Citrus aurantium amara, Cnicus benedictus, Coffea arabica, Cola nipida, Coleus barbatus, Coleus blumei, Coleus esquirolii, Coleus forskohlii, Coleus scutellaroides, Coleus sp., Coleus xanthantus, Commiphora myrrha, Crithmum maritimum, Cuminum cyminum, Dioscorea colettii, Dioscorea villosa, Eugenia caryophyllus, Filipendula ulmaria L, Foeniculum vulgare,* foenum-graecum, *Fucus vesiculosus, Ginkgo biloba,* ginkgo biloba, *Glycine max, Glycyrrhiza glabra, Hedera helix* (ivy extract), *Hibiscus sabdariffa, Hordeum vulgare, Humulus lupulus, Hyperycum perforatum, Ilex paraguariensis, Kigelia africana, Laminaria digitata, Lilium brownii, Lupinus perennis, Lycopersicon pimpinellifolium, Medusomyces gisevi, Nelumbium speciosum, Nicotianum tabacum, Orthosiphon stamincus benth, Panax ginseng, Paullinia cupana, Peumus boldus, Phyllacantha fibrosa, Piper methysticum, Piper nigrum, Prunella vulgaris, Prunus amygdalus dulcis, Quillaja saponaria, Radix asparagi, Radix sarsaparrilla, Rosmarinus officinalis, Rubus idaeus, Ruscus aculeatus* (extract of Butcher's broom), *Salvia officinalis L, Sambucus nigra, Serenoa repens, Smilax aristolochiaefolia, Smilax aspera* (rough bindweed), *Smilax ornata, Solanum paniculatum, Spirulina platensis algae, Taraxacum erythrospermum, Taraxacum officinale,* green tea, Tian-dong, *Tribulus terrestri, Trifolium pratense, Trifolium repens, Trigonella foenum graecum, Turnera diffusa, Ulmus rubra, Uncaria tomentosa, Verbena officinalis, Vitex agnus-castus, Yuca spp, Yucca brevifolia, Yucca filamentosa, Yucca filifera, Yucca schudufera, Yucca vaccata* or zhi-mu among others, alverin, alverin citrate, dihydromyricetin, coenzyme A, lipase, cerulenin, sirtuin, rutin, glaucine, esculin, visnadine, caffeine, theophylline, theobromine, aminophylline, xanthine, carnitine, forskolin, escin, ruscogenin, hederin, triethanolamine iodide, sarsasapogenin, parigenin, smilagenin, isosarsasapogenin, epi-tigogenin, tigogenin, epi-sarsasapogenin, neotigogenin, epi-smilagenin, parillin, timosaponin, xilingsaponin, filiferin, AMPc synthesis inducing or inhibiting agents, Lanachrys® [INCI: Chrysanthellum Indicum Extract] marketed by Atrium/Unipex, Slim-Excess™ [INCI: Water, Butylene Glycol, Sodium Chloride, Hydrolyzed Carrageenan, Xanthan Gum], Sveltine™ [INCI: Water, Butylene Glycol, Carnitine, Lecithin, Caffeine, Carbomer, Salicylic Acid, Atelocollagen, Centella Asiatica Extract, Esculin, Sodium Chondroitin Sulfate], Peru Liana [INCI: Uncaria Tomentosa Extract] or Flavenger™ [INCI: Caprylic/Capric Triglyceride, Silica Dimethyl Silylate, Glyceryl Oleate, Quercetin Caprylate] marketed by BASF, Scopariane [INCI: Sphacelaria Scoparia], Phyco R75 [INCI: Laminaria Digitata], Pheoslim [INCI: Phyllacantha Fibrosa Extract], Buckwheat Wax [INCI: Polygonum fagopyrum] or Areaumat Samphira [INCI: Crithmum Maritimum Extract] marketed by Codif, Slimming Factor Karkade™ [INCI: Hibiscus Sabdariffa] marketed by Cosmetochem, Liposuctionine [proposed INCI: Acetyl Hexapeptide] marketed by Infinitec Activos, Xantalgosil C® [INCI: Acefylline Methylsilanol Mannuronate], Theophyllisilane C® [INCI: Methylsilanol Carboxymethyl Theophylline Alginate] or Glutrapeptide® [INCI: Pyroglutamylamidoethyl Indole] marketed by Exsymol, Timiline® [INCI: Polyglucuronic acid] or Kigeline® [INCI: Kigelia Africana Extract] marketed by Greentech, Visnadine [INCI: Visnadine] or Ginkgo Biloba Dimeric Flavonoids Phytosome [INCI: Phospholipids, Ginkgo Biloba Leaf Extract] marketed by Indena, Slimfit® LS 9509 [INCI: Cecropia Obtusifolia Bark Extract] marketed by Laboratoires Serobiologiques/Cognis, Silusyne™ [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39], Liporeductyl® [INCI: Water, Glycerin, Lecithin, Caffeine, Butcherbroom (Ruscus Aculeatus) Root Extract, Maltodextrin, Silica, Tea-Hydroiodide, Propylene Glycol, Ivy (Hedera Helix) Extract, Carnitine, Escin, Tripeptide-1, Xanthan Gum, Carrageenan (Chondrus Crispus), Disodium EDTA] (Water, Glycerin, Lecithin, Caffeine, Extract of Butcher's Broom root (Ruscus Aculeatus), Maltodextrin, Silica, Triethanolamine Hydroiodide, Propylen Glycol, Ivy Extract (Hedera Helix), Carnitine, Escin, Tripeptide-1, Xanthan Gum, Carrageenan (Chondrus Crispus), EDTA (Disodium) marketed by Lipotec, Iso-Slim Complex [INCI: Soy Isoflavones, Caffeine, Carnitine, Spirulina Platensis Extract, Polysorbate 80, Alcohol, Phenoxyethanol, Aqua], Happybelle-PE [INCI: Lecithin, Vitex Agnus Castus Extract, Glycerin, Ascorbyl Tetraisopalmitate, Tocopherol, Caprylic/Capric Triglyceride, Cyclodextrin, Alcohol, Water] or AmaraShape [INCI: Lecithin, Caffeine, Citrus Aurantium Amara Extract, Pentylene Glycol, Alcohol, Water] marketed by Mibelle Biochemistry, Regu®-Slim [INCI: Maltodextrin, Caffeine, Paullinia Cupana Seed Extract, Carnitine, Microcrystalline Cellulose, Cysteic Acid, Pantheine Sulfonate] or Regu®-Shape [INCI: Isomerized Linoleic Acid, Lecithin, Glycerin, Polysorbate 80] marketed by Pentapharm/DSM, Voluplus™ [INCI: Macadamia Ternifolia Seed Oil, Macelignan, Tocopherol], Provislim™ [INCI: Propanediol, Water (Aqua), Fisetin, Raspberry Ketone], Noline [INCI: Macelignan (Myristica fragans)], Myriceline [INCI: Dihydromyricetin] or Drenalip [INCI: Ruscus Aculeatus Root Extract, Citrus Medica Limonum Peel Extract, Solidago Virgaurea Extract, Astragalus Membranaceus Root Extract] marketed by Provital, Actisculpt [INCI: Commiphora Myrrha Extract, Coleus Forskohlii Root Extract] marketed by Quest, Perfeline® [INCI: Water, Carnitine, Caffeine, Ruscus Aculeatus Extract], CellActive® Shape [INCI: Chlorella Vulgaris/Lupinus Albus Protein Ferment, Coleus Forskohlii, Caffeine] or CellActive® Form [INCI: Garcinia Mangostana Peel Extract, Chlorella Vulgaris/Lupinus Albus Protein Ferment, Pyrus Cydonia Seed Extract] marketed by Rahn, ProContour™ [INCI: Water, Alcohol, Lecithin, Caffeine, Carnitine, Centella Asiatica Leaf Extract, Potassium Phosphate, Coleus Forskohlii Root Extract] marketed by Rovi Cosmetics, Volufiline™ [INCI: Anemarrhenae asphodeloides (root) extract], Unislim™ [INCI: Ilex Paraguariensis (Leaf) Extract, Water, Butylene Glycol, Coffea Arabica (Coffee) Seed Extract (Bean), PEG-60 Almond Glycerides, Glycerin, Cetyl Hydroxyethylcellulose], Redulite™ [INCI: Glycerin, Aqua, Ethoxydiglycol, Sambucus Nigra, Sodium Polyacrylate], Pleurimincyl™ [INCI: Caffeine, Bupleurum Chinensis extract], Phytotal™ SL [INCI: Glycerin, Verbena Officinalis Extract, Butylene Glycol, Sambucus Nigra Flower Extract, Eugenia Caryophyllus (Clove) Flower Extract, Lecithin], Phytosonic™ [INCI: Aqua, Euglena Gracilis Extract, Caffeine, Glaucium Flavum Leaf Extract], Ovaliss™ [INCI: Glycerin, Aqua, Coco-glucoside, Caprylyl Glycol, Alcohol, Glaucine], Lipocare™ [INCI: Caffeine, Coenzym A, Bupleurum Chinensis extract], Cyclolipase™ [INCI: Glyceryl Polymethacrylate, Water, Caffeine, Lipase, Aden-Adenosine Phosphate], Coaxel™ [INCI: Caffeine, Coenzyme A, Carnitine, Water, Glycerin] or Bodyfit™ [INCI: Glycerin, Aqua (Water), Coco-Glucoside, Caprylyl Glycol, Alcohol, Glaucine] marketed by Sederma/Croda, Voluform [INCI: Palmitoyl isoleucine], Adiposlim [INCI: Sorbitan Laurate, Lauroyl Proline] or Adipoless [INCI: Butylene Glycol, Chenopodium Quinoa Seed Extract] marketed by Seppic, Slimactive® [INCI: Peumus Boldus Leaf Extract], Remoduline® [INCI: Citrus Aurantium Amara Flower Extract], Pro-Sveltyl [INCI: Nelumbium Speciosum Extract], Biosculptine® [INCI: Hydrolyzed Celosia Cristata Flower/Seed Extract, Hydrolyzed Prunella Vulgaris Extract] or Affiness® [INCI: Hydrolyzed Coriandrum Sativum Fruit Extract, Citrus Aurantium Dulcis (Orange) Fruit Extract] marketed by Silab, Delipidol [INCI: Tyrosyl Punicate], Guaraslim® [INCI: Butylene Glycol, Water, Caffeine, Paullinia Cupana Seed Extract, Ptychopetalum Olacoides Bark Extract] or Caobromine® [INCI: Theobroma Cocoa Shell Extract] marketed by Solabia, Abdoliance [INCI: Sucrose palmitate, Polysorbate 20, Glyceryl Linolenate, Paullinia Cupana Seed Extract, Maltodextrin, Prunus Amygdalus Dulcis (Sweet Almond) Oil, Lecithin, Water, Citrus Aurantium Amara (Bitter Orange) Peel Extract, Phenoxyethanol, Tocopherol], Betaphroline [INCI: Tephrosia Purpurea Seed Extract] or Commipheroline [INCI: Commiphora Mukul Resin Extract] marketed by Soliance, UCPeptide™ V [INCI: Water, Butylene Glycol, Pentapeptide] or ATPeptide™ IS [INCI: Tripeptide-3] marketed by Vincience/ISP among others, or mixtures thereof.

In one embodiment, the firming and/or redensifying and/or restructuring agent is selected, for example and not restricted to, from the group formed by extracts of *Malpighia punicitolia, Cynara scolymus, Gossypium herbaceum, Aloe Barbadensis, Panicum miliaceum, Morus nigra, Sesamum indicum, Glycine soja, Triticum vulgare,* Pronalen^{®} Refirming HSC [INCI: Triticum Vulgare, Silybum Marianum, Glycine Soy, Equisetum Arvense, Alchemilla Vulgaris, Medicago Sativa, Raphanus Sativus] or Polyplant^{®} Refirming [INCI: Coneflower, Asiatic Centella, Fucus, Fenugreek] marketed by Provital, Lanablue^{®} [INCI: Sorbitol, Algae Extract] marketed by Atrium Biotechnologies/Unipex Innovations, Pepha^{®}-Nutrix [INCI: Natural Nutrition Factor] marketed by Pentapharm/DSM, plant extracts containing isoflavones, Biopeptide EL™ [INCI: Palmitoyl Oligopeptide], Biopeptide CL™ [INCI: Palmitoyl Oligopeptide], Vexel^{®} [INCI: Water (Aqua), Propylene Glycol, Lecithin, Caffeine, Palmitoyl Carnitine], Matrixyl^{®} [INCI: Palmitoyl Pentapeptide-3], Matrixyl^{®} 3000 [INCI: Palmitoyl Tetrapeptide-3, Palmitoyl Oligopeptide] or Bio-Bustyl™ [INCI: Glyceryl Polymethacrylate, Rahnella Soy Protein Ferment, Water (Aqua), Propylene Glycol, Glycerin, PEG-8, Palmitoyl Oligopeptide] marketed by Sederma/Croda, Dermosaccharides^{®} HC [INCI: Glycerin, Water (Aqua), Glycosaminoglycans, Glycogen], Aglycal^{®} [INCI: Mannitol, Cyclodextrin, Glycogen, Aratostaphylos Uva Ursi Leaf Extract], Cytokinol^{®} LS [INCI: Hydrolyzed Casein, Hydrolyzed Yeast Protein, Lysine HCl] or Firmiderm^{®} LS9120 [INCI: Terminalia Catappa Leaf Extract, Sambucus Negra Flower Extract, PVP, Tannic Acid] marketed by Laboratoires Serobiologiques/Cognis/BASF, Liftline^{®} [INCI: Hydrolyzed Wheat Protein], Raffermine^{®} [INCI: Hydrolyzed Soy Flour] or Ridulisse C^{®} [Hydrolyzed Soy Protein] marketed by Silab, Serilesine^{®} [INCI: Hexapeptide-10], Decorinyl™ [INCI: Tripeptide-10 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Silusyne™ [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39] or Adifyline™[INCI: Acetyl Hexapeptide-38] marketed by Lipotec/Lubrizol, Ursolisome^{®} [INCI: Lecithin, Ursolic Acid, Atelocollagen, Xanthan Gum, Sodium Chondroitin Sulfate] or Collalift^{®} [INCI: Hydrolyzed Malt Extract] marketed by Coletica/Engelhard/BASF, Syn^{®}-Coll [INCI: Palmitoyl Tripeptide-5] marketed by Pentapharm/DSM, Hydriame^{®} [INCI: Water (Aqua), Glycosaminoglycans, Sclerotium Gum] marketed by Atrium Biotechnologies/Unipex Innovations or IP2000 [INCI: Dextran, Trifluoroacetyl Tripeptide-2] marketed by Institut Europeen de Biologie Cellulaire/Unipex Innovations, among others.

In one embodiment, the agent stimulating the synthesis of dermal or epidermal macromolecules is selected, for example and not restricted to, from the group formed by collagen synthesis-stimulating agents, elastin synthesis-stimulating agents, decorin synthesis-stimulating agents, laminin synthesis-stimulating agents, chaperone synthesis-stimulating agents, sirtuin synthesis-stimulating agents, sirtuin activating agents, aquaporin synthesis-modulating agents, fibronectin synthesis-stimulating agent, agents that inhibit collagen degradation, agents that inhibit elastin degradation, agents that inhibit serine proteases such as kallikreins, leukocyte elastase or cathepsin G, agents stimulating fibroblast proliferation, and DNA repairing agents and/or DNA protecting agents, such as and not restricted to extracts of *Centella asiatica, Saccharomyces cerivisiae, Solanum tuberosum, Rosmarinus officinalis, Vaccinium angustifolium,* extract of the algae *Macrocystis pyrifera, Padina pavonica,* extract of soy, malt, flax, sage, red clover, kakkon, white lupin plants, hazelnut extract, maize extract, yeast extract, beech shoot extracts, leguminous seed extract, plant hormone extract such as gibberellins, auxins or cytokinins, among others, or extract of saline zooplankton, the fermentation product of milk with *Lactobacillus Bulgaricus,* asiaticosides and their derivatives, vitamin C and its derivatives, cinnamic acid and its derivatives, Matrixyl^{®} [INCI: Palmitoyl Pentapeptide-3], Matrixyl^{®} 3000 [INCI: Palmitoyl Tetrapeptide-3, Palmitoyl Oligopeptide] or Biopeptide CL™ [INCI: Glyceryl Polymethacrylate, Propylene Glycol, Palmitoyl Oligopeptide] marketed by Sederma/Croda, Antarcticine^{®} [INCI: Pseudoalteromonas Ferment Extract], Decorinyl^{®} [INCI: Tripeptide-10 Citrulline], Serilesine^{®} [INCI: Hexapeptide-10], Lipeptide [INCI: Hydrolized Vegetable Protein], Aldenine^{®} [INCI: Hydrolized Wheat Protein, Hydrolized Soy Protein, Tripeptide-1], Relistase™ [INCI: Acetylarginyltriptophyl Diphenylglycine], Thermostressine™ [INCI: Acetyl Tetrapeptide-22], Peptide AC29 [INCI: Acetyl Tripeptide-30 Citrulline], Diffuporine™ [INCI: Acetyl Hexapeptide-37], Silusyne™ [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39] or Adifyline™[INCI: Acetyl Hexapeptide-38] marketed by Lipotec/Lubrizol, Drieline^{®} PF [INCI:Yeast Betaglucan] marketed by Alban Muller, Phytovityl C^{®} [INCI: Aqua, Zea Mays Extract] comercializado por Solabia, Collalift^{®} [INCI: Hydrolyzed Malt Extract] marketed by Coletica/Engelhard/BASF, Phytocohesine PSP™ [INCI: Sodium Beta-Sitosterol Sulfate] marketed by Vincience/ISP/Ashland, minerals such as calcium, among others, retinoids and their derivatives, isoflavonoids, carotenoids, such as lycopene, pseudodipeppseudodipeptides, retinoids and their derivatives such as retinol or retinyl palmitate, among others, or heparinoids, among others.

In one embodiment, the elastin synthesis-stimulating agent or agent that inhibit elastin degradation is selected, for example and not restricted to, from the group formed by Elhibin^{®} [INCI: Glycine Soja (Soybean) Protein], Preregen^{®} [INCI: Glycine Soja (soybean) Protein, Oxido Reductases] or Regu^{®}-Age [INCI:Hydrolyzed Rice Bran Protein, Glycine Soja (Soybean) Protein, Oxido Reductases] marketed by Pentapharm/DSM, Juvenesce [INCI: Ethoxydiglicol and caprylic Triglycerid, Retinol, Ursolic Acid, Phytonadione, llomastat], Micromerol™ [INCI: Pyrus Malus Extract], Heather Extract [INCI: Calluna Vulgaris Extract], Extracellium^{®} [INCI: Hydrolyzed Potato Protein] or Flavagrum™ PEG [INCI: PEG-6 Isostearate, Hesperetin Laurate] marketed by Coletica/Engelhard/BASF, Proteasyl^{®} TP LS8657 [INCI: Pisum Sativum Extract] marketed by Laboratoires Sérobiologiques/Cognis, acetyl-arginyl-phenylglycyl-tryptophyl-phenylglycine, acetyl-arginyl-phenylglycyl-valyl-glycine or acetyl-arginyl-phenylglycyl-valyl-phenylglycine marketed by Lipotec, Sepilift DPHP [INCI: Dipalmitoyl hydroxyproline] marketed by SEPPIC, Vitaderm^{®} [INCI: Alcohol, Water, Glycerin, Hydrolyzed Rice Protein, Ilex Aquifolium Extract, Sodium Ursolate, Sodium Oleanolate] marketed by Rahn, Gatuline^{®} Age Defense 2 [INCI: Juglans Regia (Walnut) Seed Extract] marketed by Gattefosse, IP 2000 [INCI: Dextran, Trifluoroacetyl Tripeptide-2] marketed by IEB and Atrium, Radicaptol [INCI: Propylene Glycol, Water, Passiflora Incarnata Flower Extract, Ribes Nigrum (Blackcurrant) Leaf Extract, Vitis Vinifera (grape) Leaf Extract] marketed by Solabia or ViaPure™ Boswellia [INCI: Olivanum (Boswellia Serrata) Extract] marketed by Soliance, among others.

In one embodiment, the humectant or substance that retains moisture, moisturizer or emollient is selected, for example and not restricted to, from the group formed by, polyols and polyethers such as glycerin, ethylhexylglycerin, caprylyl glycol, pentylene glycol, butylene glycol, propylene glycol and its derivatives, triethylene glycol, polyethylene glycol, Glycereth-26, Sorbeth-30; panthenol; pyroglutamic acid and its salts or derivatives; amino acids, such as serine, proline, alanine, glutamate or arginine; ectoin and its derivatives; *N*-(2-hydroxyethyl)acetamide; pyrrolidone carboxylic acid (PCA); *N-*lauroyl-pyrrolidone carboxylic acid; *N*-lauroyl-L-lysine; *N*-alpha-benzoyl-L-arginine; urea; creatine; alpha- and beta-hydroxy acids such as lactic acid, glycolic acid, malic acid, citric acid, tartaric acid or salicylic acid, and its salts; polyglyceryl acrilate; sugars and polysaccharides, such as glucose, isomerate saccharide, sorbitol, pentaerythritol, inositol, xylitol, sorbitol, trehalose and its derivatives, sodium glucuronate, carrageenans *(Chondrus crispus)* or chitosan; glycosaminoglycans such as hyaluronic acid and its derivatives; aloe vera in any of its forms; honey; soluble collagen; lecithin and phosphatidylcholine; ceramides; cholesterol and its esters; tocopherol and its esters, such as tocopheryl acetate or tocopheryl linoleate; long chain alcohols such as cetearyl alcohol, stearic alcohol, cetyl alcohol, oleyl alcohol, isocetyl alcohol or octadecan-2-ol; long chain alcohol esters such as lauryl lactate, myristyl acetate or C₁₂-C₁₅ alkyl benzoates; fatty acids such as stearic acid, isostearic acid or palmytic acid; polyunsaturated fatty acids (PUFAs); sorbitans such as sorbitan distearate; glycerides such as glyceryl monoricinoleate, glyceryl monostearate, glyceryl stearate citrate or caprylic and capric acid triglyceride; saccharose esters such as saccharose palmitate or saccharose oleate; butylene glycol esters, such as dicaprylate and dicaprate; fatty acid esters such as isopropyl isostearate, isobutyl palmitate, isocetyl stearate, isopropyl laurate, hexyl laurate, decyl oleate, cetyl palmitate, di-n-butyl sebacate, isopropyl myristate, isopropyl palmitate, isopropyl stearate, butyl stearate, butyl myristate, isopropyl linoleate, 2-ethylhexyl palmitate, 2-ethylhexyl cocoate, decyl oleate, myristyl myristate; squalene; mink oil; lanolin and its derivatives; acetylated lanolin alcohols; silicone derivatives such as cyclomethicone, dimethicone or dimethylpolysiloxane; Diffuporine™ [INCI: Acetyl Hexapeptide-37], Antarcticine^{®} [INCI: Pseudoalteromonas Ferment Extract], Xpertmoist™ [INCI: Glycerin, Pseudoalteromonas Ferment Extract, Xanthan Gum, Proline, Alanine, Serine, Ethylhexylglycerin, Caprylyl Glycol], Bodyfensine™ [INCI: Acetyl Dipeptide-3 Aminohexanoate], Hyanify™ [INCI: Saccharide Isomerate] or HyadisineTM [INCI: Pseudoalteromonas Ferment Extract] marketed by Lipotec/Lubrizol; petrolatum; mineral oil; mineral and synthetic waxes; beeswax (cera alba); paraffin; or waxes and oils of plant origin such as candelilla wax *(Euphorbia cerifera),* carnauba wax *(Copernicia cerifera),* shea butter *(Buti-(Butirospermum parkii),* cocoa butter *(Theobroma cacao),* castor oil *(Ricinus communis),* sunflower oil *(Helianthus annuus),* olive oil *(Olea europaea),* coconut oil *(Cocos nucifera),* palm oil *(Elaeis guineensis),* wheat germ oil *(Triticum vulgare),* sweet almond oil *(Prunus amygdalus dulces),* musk rose seed oil *(Rosa moschata),* wild soybean oil *(Glycine soja*), grape seed oil *(Vitis vinifera),* calendula oil *(Calendula officinalis),* jojoba oil *(Simmonsis chinensis),* mango oil *(Mangifera indica),* avocado oil *(Persea gratissima)* among others, and/or mixtures thereof.

In one embodiment, the cosmetic and/or absorbent and/or body odor masking deodorant and/or antiperspirant agent, perfuming substance and/or perfumed oil is selected, for example and not restricted to, from the group formed by the complex zinc salt of ricinoleic acid, Styrax, derivatives of abiotic acid, sage essence, chamomile essence, carnation essence, lemon balm essence, mint essence, cinnamon leaf essence, lime flower essence, juniper berry essence, vetiver essence, olibanum essence, galbanum essence, labdanum essence, lavender essence, peppermint essence, bergamot orange, dihydromyrcenol, lilial, lyral, citronellol, lemon essence, mandarin essence, orange essence, lavender essence, muscat, geranium bourbon essence, aniseed, cilantro, cumin, juniper, extracts of fleur-de-lis, lilac, roses, jasmin, bitter orange blossom; benzyl acetate, *p-tert*-butylcyclohexyl acetate, linalyl acetate, phenylethyl acetate, ethylmethylphenyl glycinate, linalyl benzoate, benzyl formate, allyl cyclohexyl propionate, styrallyl propionate, benzyl salicylate, benzyl ethyl ether, linear alkanes with from 8 to 18 carbon atoms, citral, ricinoleic acid, citronellal, citronellyl oxyacetaldehyde, cyclamen aldehyde, hydroxycitronellal, bourgeonal, ionones, methyl cedryl ketone, anethole, eugenol, isoeugenol, geraniol, linalool, terpineol, phenylethyl alcohol, α-hexylcinnamaldehyde, geraniol, benzylacetone, cyclamen aldehyde, Boisambrene Forte^{®}, ambroxan, indole, hedione, sandelice, cyclovertal, β-damascone, allyl amyl glycolate, dihydromyrcenol, phenoxyethyl isobutyrate, cyclohexyl salicylate, Vertofix Coeur, Iso-E-Super, Fixolide NP, Evernyl, phenylacetic acid, geranyl acetate, romillat, irotyl, floramate, active astringent products such as aluminum chloride, aluminum chlorohydrate, aluminum dichlorohydrate, aluminum sesquichlorohydrate, aluminum hydroxyallantoinate, aluminum chlorotartrate, aluminum and zirconium trichlorohydrate, aluminum and zirconium tetrachlorohydrate, aluminum and zirconium pentachlorohydrate and/or mixtures thereof.

In one embodiment, the agent inhibiting sweat-degrading enzymes is selected, for example and not restricted to, from the group formed by trialkyl citrates such as trimethyl citrate, tripropyl citrate, triisopropyl citrate, tributyl citrate or triethyl citrate; lanosterine sulfate or phosphate, cholesterin, campesterin, stigmasterin and sitosterin; dicarboxylic acids and their esters, such as glutaric acid, monoethyl glutarate, diethyl glutarate, adipic acid, monoethyl adipate, diethyl adipate; malonic acid and diethyl malonate, hydroxycarboxylic acids and their esters such as malic acid, tartaric acid or diethyl tartrate, zinc glycinate and/or mixtures thereof.

In one embodiment, the whitening or depigmenting agent or melanin synthesis inhibiting agent, is selected, for example and not restricted to from the extracts of *Achillea millefolium, Aloe vera, Aradirachta indica, Asmuna japonica, Autocarpus incisus, Bidens pilosa, Broussonetia papyrifera, Chlorella vulgaris, Cimicifuga racemosa, Emblica officinalis, Glycyrrhiza glabra, Glycyrrhiza uralensis, Ilex purpurea, Ligusticum lucidum, Ligusticum wallichii, Mitracarpus scaber, Morinda citrifolia, Morus alba, Morus bombycis, Naringi crenulata, Prunus domesticus, Pseudostellariae radix, Rumex crispus, Rumex occidentalis, Sapindus mukurossi, Saxifragia sarmentosa, Scutellaria galericulate, Sedum sarmentosum bunge, Stellaria medica, Triticum Vulgare, Arctostaphylos Uva ursi* or *VVhitania somnifera* among others and/or Lipochroman™ [INCI: Dimethylmethoxy Chromanol], Chromabright^{®} [INCI: Dimethylmethoxy Chromanyl Palmitate] marketed by Lipotec/Lubrizol, Whitami [INCI: Maltodextrin, Papain, Titanium Dioxide, Angelica Acutiloba Root Extract, Saposhnikovia Divaricata Root Extract, Thioctic Acid, Kaolin, Ascorbyl Glucoside, Pinus Pinaster Bark Oligomeric Proanthocyanidins] marketed by Alban Muller; NAB^{®} Asafetida Extract [INCI: Aqua (Water), Butylene Glycol, Ethoxydiglycol, Ferula Foetida Extract] marketed by Arch; Licorice Roots Extract [INCI: Licorice (Glycyrrhiza Glabra) Extract] marketed by Campo Research; Betides™ [INCI: Bellis Perennis (Daisy) Flower Extract] marketed by CLR; Algowhite [INCI: Ascophyllum Nodosum Extract] marketed by Codif; Biowhite™ [INCI: Saxifraga Sarmentosa Extract, Vitis Vinifera (Grape) Fruit Extract, Butylene Glycol, Water, Morus bombycis Root Extract, Scutellaria Baicalensis Root Extract, Disodium EDTA], Melarrest^{®} A [INCI: Glycerin, Lactic Acid, Kojic Acid, Ascorbic Acid], Melarrest^{®} L [INCI: Water, Cyclopentasiloxane, Butylene Glycol, Propylene Glycol, Phospholipids, Glycyrrhiza Glabra (Liquorice) Extract, Kojic Acid, Ammonium Glycyrrhizate], Vitagen [INCI: Aminopropyl Ascorbyl Phosphate] or Collalift [INCI: Hydrolyzed Malt Extract], marketed by Coletica/Engelhard/BASF; DC Skin Bright™ [INCI: PEG-12 Glyceryl Distearate, Methyl Dihydroxybenzoate, Ethoxydiglycol, Polyethylene, Water] marketed by DC Ingredients; DS-WHITEKLE [INCI: Acetylphytosphingosine] marketed by Doosan; TEGO Cosmo C 250 [INCI: 1-methylhydantoine-2-imide] and TEGO Pep 4-Even [INCI: Glycerin, Tetrapeptide-30] marketed by Evonik Goldschmidt; Albatin^{®} [INCI: Aminoethylphosphinic Acid, Butylene Glycol, Water] marketed by Exsymol; Synerlight™ [INCI: Actinidia Chinensis (Kiwi) Fruit Water, Butylene Glycol, Alcohol, Sophora Angustifolia Root Extract] marketed by Gattefossé; Clerilys™ [INCI: Water, Cucumis Santivus, Morus Alba Extract, Hibiscus Sabdariffa Extract, Wine Extract] marketed by Greentech; Melanostatine^{®}-5 [INCI: Dextran, Nonapeptide-1] marketed by IEB/Unipex; Actiwhite™ [INCI: Water, Glycerin, Sucrose Dilaurate, Polysorbate 20, Pisum Sativum Extract], Active^{®} Powder Whiteness [INCI: Water, Lauryl Methacrylate/Glycol Dimethacrylate Copolymer, Butylene Glycol, Dicaprylyl Ether, Titanium Dioxide, Algae, Citric Acid, Sodium Citrate, Waltheria Indica Leaf Extract, Ferulic Acid, Polyglyceryl-2-Dipolyhydroxystearate], Dermawhite^{®} NF LS 9410 [INCI:Mannitol, Sodium Gluconate, Citric Acid, Sodium Citrate, Waltheria Indica Leaf Extract, Dextrin, Ferulic Acid], Radianskin™ [INCI:Hydroxyphenoxy Propionic Acid] marketed by L. Serobiologiques/Cognis/BASF; Lipobrite^{®} HCA-4 [INCI:PEG-4, Hydroxycinnamic Acid] marketed by Lipochemicals; Whitessence™ [INCI:Artocarpus Heterophyllus Seed Extract, Maltodextrin, Disodium Phosphate, Sodium Phosphate] marketed by Lucas Meyer; Emblica™ [INCI:Phyllanthus Emblica Fruit Extract] marketed by Merck; SulforaWhite [INCI:Lepidium Sativum Sprout Extract, Glycerin, Lecithin, Phenoxyethanol, Aqua], Delentigo™ [INCI:Lepidium Sativum Sprout Extract, Lecithin, Soy Isofla-Isoflavones, Polysorbate 80, Alcohol, Glycerin, Phenoxyethanol, Water] marketed by Mibelle; Alpha-Arbutin [INCI:Alpha-arbutin], Gigawhite [INCI:Water, Glycerin, Malva Sylvestris (Mallow) Extract, Mentha Piperita Leaf Extract, Primula Veris Extract, Alchemilla Vulgaris Extract, Veronica Officinalis Extract, Melissa Officinalis Leaf Extract, Achillea Millefolium Extract], Melawhite^{®} [INCI: Leukocyte Extract, AHA], Melfade^{®}-J [INCI:Water, Arctostaphylos Uva-Ursi Leaf Extract, Glycerin, Magnesium Ascorbyl Phosphate] or Regu-Fade [INCI: Resveratrol] marketed by Pentapharm/DSM; CellActive^{®} White [INCI: Aqua, Alcohol denat., Niacinamide, Zinc PCA, Chlorella Vulgaris/Lupinus Albus Protein Ferment, Nasturtium Officinale Extract] Illumiscin^{®} [INCI:Glycerin, Aqua (Water), Olea Europaea Leaf Extract, Ascorbyl Glucoside, Zinc PCA] marketed by Rahn; Arlatone™ Dioic DCA [INCI: Octadecenedioic Acid, BHT], Etioline™ [INCI:Glycerin, Butylene Glycol, Arctostaphylos Uva Ursi Leaf Extract, Mitracarpus Scaber Extract], Lumiskin™ [INCI:Caprylic/Capric Triglycerid, DiacetylBoldine], Melaclear™ 2 [INCI:Glycerin, Water, Dithiaoctanediol, Gluconic Acid, Sutilains, Beta-carotene], Lumisphere™ [INCI:Water (Aqua), Titanium Dioxide, Polysorbate 20, Cetyl Hydroxyethylcellulose, Polymethylmethacrylate, Trilaurin, Diacetyl boldine], O.D.A.white™ [INCI:Octadecenedioic Acid], Wonderlight™ [INCI:Humulus Lupulus (Hops) Strobile] marketed by Sederma/CRODA; Sepiwhite™ MSH [INCI:Undecylenoyl phenylalanine], Sepicalm™ VG [INCI:Sodium palmitoyl proline, Nymphaea Alba Flower Extract] marketed by Seppic; Clariskin II [INCI:Triticum Vulgare Extract], Dermalight^{®} [INCI:Tropaeolum Majus Extract], Whitonyl^{®} [INCI:Palmaria Palmata Extract] marketed by Silab; DermaPep A350 [INCI: Myristol Tripeptide-31, Butylene Glycol] or DermaPep W411 [INCI: Palmitoyl Hexapeptide-36, Methyl Undecenoyl Leucinate, Butylene Glycol] marketed by Dermapep, Neurolight.61G [INCI: Glycerin, Water, Pancratium Maritimum Extract] marketed by Codif, Azeloglicina^{®} [INCI:Potassium Azelaoyl Diglycinate] marketed by Sinerga; Whitesphere Premium [INCI:Sucrose Palmitate, Butylene Glycol , Glyceryl Linoleate, Prunus Amygdalus Dulcis, Almond Oil, Water (aqua), Glycyrrhiza Glabra (Liquorice) Root Extract, Magnesium Ascorbyl Phosphate, Undaria Pinnatifida Extract], Axolight [INCI:Triticum Aestivum Extract] marketed by Soliance; SymWhite^{®} [INCI:Phenylethyl Resorcinol], Extrapone™ Nutgrass GW [INCI:Cyperus Rotundus Root Extract] marketed by Symrise; Synovea® HR [INCI:Hexylresorcinol] marketed by Sytheon; β-White [INCI:Water, Butylene Glycol, Hydrogenated Lecithin, Sodium Oleate, Oligopeptide-68, Disodium EDTA] marketed by Unipex; Achromaxyl™ [INCI:Brassica Napus Extract] marketed by Vincience/ISP; arbutin and its isomers, kojic acid and its derivatives, vitamin C and its derivatives, for example and not restricted to, 6-O-palmitoyl ascorbic acid, dipalmitoyl ascorbic acid, magnesium salt from ascorbic-2-phosphate acid (MAP), sodium from ascorbic-2-phosphate acid (NAP), ascorbyl glucoside or ascorbyl tetraisopalmitate (VCIP) among others, retinol and its derivatives, including tretinoin and isotretinoin, idebenone, hydroxybenzoic acid and its derivatives, flavonoides, soy extract, extract of lemon, extract of orange, extract of ginkgo, extract of cucumber, extract of geranium, extract of bearberry, extract of carob, extract of cinnamon, extract of marjoram, extract of rosemary, extract of clove, soluble extract of liquorice, extract of blackberry leaf, niacinamide, liquiritin, resorcinol and its derivatives, hydroquinone, α-tocopherol, γ-tocopherol, azelaic acid, resveratrol, mercury salts, linoleic salts, α-lipoic acid, dihydrolipoic acid, alfa hydroxy acids, beta hydroxy acids, ellagic acid, ferulic acid, cinnamic acid, oleanolic acid, aloesin and its derivatives and/or inhibitors of serine protease activity, for example and not restricted to, inhibitors of tryptase, trypsin or PAR-2 activity, among others.

In one embodiment, the agent capable of filtering UV rays is selected, for example and not restricted to, from the group formed by organic or mineral photoprotective agents active against A and/or B ultraviolet rays such as substituted benzotriazoles, substituted diphenylacrylates, organic nickel complexes, umbelliferone, urocanic acid, biphenyl derivatives, stilbene, 3-benzylidene camphor, and derivatives thereof such as 3-(4-methylbenzylidene)camphor; derivatives of 4-aminobenzoic acid, 2-ethylhexyl 4-(dimethylamino)benzoate, 2-octyl 4-(dimethylamino)benzoate and amyl 4-(dimethylamino)benzoate; cinnamic acid esters, such as 2-ethylhexyl 4-methoxycinnamate or diethylamino hydroxybenzoyl hexyl benzoate, propyl 4-methoxycinnamate, isoamyl 4-methoxycinnamate, 2-ethylhexyl (octocrylenes) 2-cyano-3,3-phenyl cinnamate; salicylic acid esters, such as 2-ethylhexyl salicylate, 4-isopropylbenzyl salicylate, homomenthyl salicylate; benzophenone derivatives, such as 2-hydroxy-4-methoxybenzophenone, 2-hydroxy-4-methoxy-4'-methylbenzophenone, 2,2'-dihydroxy-4-methoxybenzophenone; benzalmalonic acid esters, such as di-2-ethylhexyl 4-methoxybenzalmalonate; triazine derivatives, such as 2,4,6-trianilino, *p*-carbo-2'-ethyl-1'-hexyloxy-1,3,5-triazine, octyl triazone or dioctyl butamido triazones; propane-1,3-diones, such as 1-(4-*tert*-butylphenyl)-3-(4'-methoxyphenyl)propane-1,3-dione; ketotricyclo(5.2.1.0)decane derivatives; 2-phenylbenzimidazole-5-sulfonic acid; benzophenone sulfonic acid derivatives, such as 2-hydroxy-4-methoxybenzophenone-5-sulfonic acid and its salts; 4-(2-oxo-3-bornylidenemethyl)benzenesulfonic acid, benzoyl methane derivatives, such as benzoyl methane 2-methyl-5-(2-oxo-3-bornylidene)sulfonic acid, such as 1-(4'-*tert*-butylphenyl)-3-(4'-methoxyphenyl)propane-1,3-dione, 4-*tert*-butyl-4'-methoxydibenzoylmethane, 1-phenyl-3-(4'-isopropylphenyl)-propane-1,3-dione, enamine compounds, anthranilates, silicons, benzimidazole derivatives, imidazolines, benzoyl derivatives, Chromabright™ [INCI: Dimethylmethoxy Chromanyl Palmitate] or Preventhelia™ [INCI: Diaminopropionoyl Tripeptide-33] both marketed by Lipotec, metal oxides such as zinc oxide, titanium, iron, zirconium, silicon, manganese, aluminum and cerium; silicates, talc, barium sulfate, zinc stearate, carbon nanotubes and/or mixtures thereof.

In one embodiment, the active ingredient is an insect repellent and is selected, for example and not restricted to, from the group formed by DEET (N,N-diethyl-meta-toluamide), ethyl hexanediol, dihydro-nepetalactone or a mixture of dihydronepetalactone stereoisomers, 3-methylbutanal, 2-methylbutanal, substituted hydroxy or amino methylbutanals; valeraldehyde and trans-pentenal, fenvalerate, esfenvalerate, 1 S,3S,4S,6R-carene-3,4-diol, dipropyl pyridine-2,5-dicarboxylate, polytetrafluoroethylene, polyvinyl fluoride and vinylidene polyfluoride, dimethylsiloxane, polyvinyl chloride, vinylidene chloride, phthalic acid, dimethyl phthalate, dibutyl phthalate, polyethylene terephthalate, indalone (butyl-3,3-dihydro-2,2-dimethyl-4-oxo-2H-pyran-6-carboxylate), curcuma *(Curcuma longa),* bicyclic iridoid lactones, iridomyrmecin pheromones, sulfur, Epsom salts (hydrated calcium sulfate), pyrethrum or Dalmatian chrysanthemum *(Chrysanthemum cinerariaefolium),* lemon grass *(Cymbopogon nardus),* linalyl acetate, 1-limonene, cineole, eugenol, eugenyl acetate, piperidines such as picaridin or bayrepel (1-piperidinecarboxylic acid, 2-(2-hydroxyethyl)-1-methylpropyl ester) and its stereoisomers, 1-(3-cyclohexen-1-yl-carbonyl)-2-methylpiperidine and its stereoisomers, or ethyl butylacetylaminopropionate, 1-(3-cyclohexene-1-yl-carbonyl)piperidine, isoiridomyrmecin, 2,3,4,5-bis(2-butylene)tetrahydrofurfural, glycol butoxy polypropylene, N-butylacetanilide, dibutyl adipate, di-n-butyl succinate, dimethyl carbate (Endo, endo)-dimethyl-bicyclo [2.2.1] hept-5-ene-2 ,3-dicarboxylate, 2-ethyl-2-butyl-1,3-propanediol, 2-ethyl-1,3-hexanediol, propyl isocinchomeronate, 2-phenylcyclohexanol, normal-propyl N,N-diethyl succinamate, butyl mesityl oxide oxalate, 2-ethyl-1,3-hexanediol, N,N-diethylbenzamide, p-methane-3,8-diol, N,N-diethylmandelamide, isopulegol hydrate, ethyl-3-[N-n-butyl-N-acetyl] aminopropionate, diisopropyl adipate, alpha-biasabal, benzyl alcohol, N,N-diethyl phenylacetamide, vitamin E, 3-acetyl-2-(2,6-dimethyl-5-heptenyl)oxazolidine (2-hydroxymethylcyclohexyl)acetic acid lactone, tefluthrin, permethrin, cypermethrin, cyhalothrin, lambda-cyhalothrin, bifenthrin, deltamethrin, achiote or annatto oil *(Bixa orellana),* lemon leaves and fruit *(Citrus medica),* bitter almond oil, anise oil, basil oil, laurel oil, caraway seed oil, cardamom oil, cedar oil, celery oil, chamomile oil, spearmint oil, cinnamon oil, citronella oil, clove oil, cilantro oil, cumin oil, dill oil, eucalyptus oil, fennel oil, ginger oil, grape oil, lemon oil, mint oil, parsley oil, catnip oil, pepper oil, rose oil, mint oil (menthol), sweet orange oil, coconut oil, cedar oil, geraniol, geranium oil, thyme oil and curcuma oil.

### Preparation of the cationic microcapsule used in the composition according to the invention

In one embodiment, the cationically charged microcapsules are obtained by a coacervation process. In another embodiment, the cationically charged microcapsules are obtained by a process where the pH of the coacervation step of the microcapsules is lower than the pH of the cross-linking step of the microcapsules, such as described in WO 2011/116962 A1. In one embodiment, the pH of the coacervation step is between 3 and 5.5, or between 4 and 5, and the pH of the cross-linking step is between 6.5 and 13, or between 7 and 10.

In one embodiment, the crosslinking agent of the cross-linking step at the coacervation process is an aldehyde such as glutaraldehyde or formaldehyde; transglutaminase, a derivative of methylenebisacrylamide, *N,N-*methylenebisacrylamide, *N,N*-(1,2- dihydroxyethylene)bisacrylamide, a derivative of ethylene glycol dimethacrylate, ethylene glycol diacrylate, diethylene glycol diacrylate, tetraethylene glycol diacrylate, ethylene glycol dimethacrylate, diethylene glycol dimethacrylate, triethylene glycol dimethacrylate, sodium tripolyphosphate, N-hydroxysuccinamide esters and/or imidoesters.

The active agent may be contained in the microcapsule in the form of a microemulsion. In one embodiment, the microemulsion is a W/O (water-in-oil) microemulsion and the active ingredient is an hydrophylic ingredient dissolved in the internal aqueous phase. In one embodiment, this microemulsion contains additionally surfactants and/or cosurfactants. In one embodiment, the surfactant of the microemulsion is selected from the group formed by nonionic surfactants, amphoteric surfactants, anionic surfactants, cationic surfactants and/or mixtures thereof. The nonionic surfactant and/or amphoteric surfactant is selected, without restriction, from the group formed by lecithins, alkyl glycosides with an alkyl group that has from 6 to 24 carbon atoms, alkylmaltosides with an alkyl group that has from 6 to 24 carbon atoms, ethoxylated alkylphenols with an alkyl group that has from 6 to 24 carbon atoms and from 5 to 30 ethylene oxide units, alkylphenol polyoxyethylene ethers with an alkyl group that has from 6 to 24 carbon atoms, saturated and unsaturated fatty alcohols with an alkyl group that has from 8 to 24 carbon atoms, poloxamers, polysorbates, fatty acid esters with sugars, sorbitane esters, polyethylene glycol fatty acid esters, castor oil, fatty alcohol and polyoxyethylene ethers, fatty acid alkanolamides, amine oxides, alkyl betaines with an alkyl group that has from 6 to 24 carbon atoms, acyl amido betaines, alkylsulfobetaines with an alkyl group that has from 6 to 24 carbon atoms, glycine derivatives, digitonin, inulin lauryl carbamate and/or mixtures thereof. In one embodiment, the nonionic surfactant and/or amphoteric surfactant is se-selected from the group formed by octyl glucoside, decyl glucoside, lauryl glucoside, octyl fructoside, dodecyl maltoside, decyl maltoside, nonoxynol-9, polyethylene glycol p-(1,1,3,3-tetramethylbutyl)phenyl ether, palmityl alcohol, oleyl alcohol, poloxamer 188, poloxamer 407, polysorbate 20, polysorbate 60, polysorbate 80, methyl glucose dioleate, sorbitan monostearate or Span 60, sorbitan monolaurate or Span 20, sorbitan monopalmitate or Span 20, sorbitan olivate, polyethylene glycol 40 stearate, polyethylene glycol 50 stearate, polyethylene glycol 100 stearate, polyoxyethylene stearyl ether, polyoxyethylene lauryl ether, cocamide monoethanolamine, cocamide diethanolamine, cocamide triethanolamine, lauramide diethanolamine, lauramide monoethanolamine, cocamidopropylamine oxide, decyl betaine, dodecyl betaine, tetradecyl betaine, cocoyl betaine, cocamidopropyl betaine, cocamidopropyl hydroxysultaine, N-2-cocoyl amidoethyl hydroxyethylglycinate and N-2-cocoyl amidoethyl hydroxyethyl carboxy glycinate and/or mixtures thereof. The anionic surfactant is selected, without restriction, from the group formed by sulfonates such as alkylbenzene sulfonates, alkyl naphthalene sulfonates, ethoxylated fatty alcohol sulfonates, aliphatic sulfonates, hydroxy alkane sulfonates, alkyl glyceryl sulfonate ethers, perfluorooctane sulfonate; alkyl sulfosuccinates, alkyl sulfoacetates; alkyl sulfates such as sodium and ammonium lauryl sulfate, ethoxylated alkyl sulfates; fatty ester sulfates; ethoxylated fatty alcohol sulfates; alkyl ether sulfates; acyl isocyanates; pentafluorooctanoates; carboxylates; ethoxylated alkylphenols; ethanol ammonium salts; diethanolammonium, methylammonium, dimethylammonium, trimethylammonium; alkyl taurates, acyl or fatty acids; alkyl or acyl sarcosinates; phosphates such as phosphate esters, alkyl phosphates, polyoxyethylene lauryl ether phosphate; glutamates; stearates; biliary acids and their salts, such as glycocholic acid and sodium glycocholate, taurococholic acid and sodium taurocholate, taurodesoxycholate and/or mixtures thereof. The cationic surfactant is selected, without restriction, from the group formed by quaternary ammonium salts, such as cetyl trimethyl ammonium bromide, lauryl trimethyl ammonium chloride, benzyl dimethyl hexadecyl ammonium chloride, distearyl dimethyl ammonium chloride, dilauryl dimethyl ammonium chloride, dimyristyl dimethyl ammonium chloride, cetylpyridinium chloride, benzalkonium chloride, benzethonium chloride, methyl benzetonium chloride and/or mixtures thereof.

### Compound (b)

The composition according to the invention further comprises water as compound (b). Preferably, distilled or deionized water may be used. In one embodiment, also pipe water may be used.

The use of water effects that the composition according to the invention may be provided in form of an aqueous dispersion.

If necessary, said water may contain organic solvents which preferably are miscible or at least partially miscible with water. Suitable solvents are preferably alcohols and glycols.

### Compound (c)

The composition according to the invention further comprises at least one non-ionic oxygen-containing compound comprising at least one alkylene group as compound (c).

Without being bound by theory, it is believed that compound (c) has the function of a dispersant in the composition according to the invention.

Preferably, said at least one compound (c) is a polyalkylene glycol ether.

The term *"polyalkylene glycol ether"* encompasses *"fatty alcohol polyglycol ethers"* and *"fatty alcohol ethoxylate (FAEO)".*

Polyalkylene glycol ethers are known or may be prepared according to known methods, preferably by reaction of an alcohol with ethylene oxide or propylene oxide or ethylene oxide and propylene oxide. Block copoylmers are also possible.

Preferably, an alcohol having from 6 to 26 carbon atoms in the carbon chain may be used, further preferably a fatty alcohol having from 6 to 22 carbon atoms.

In one embodiment, said polyalkylene glycol ether is of formula (i)

R₁-O-(CH₂-CHR₂)ₙ-OH (i)

wherein R₁ is a branched or unbranched alkyl or alkenyl group having from 6 to 26 carbon atoms; and R₂ is H or CH₃; or said ether of formula (i) comprises moieties in which is R₂ is H and mioieties in which R₂ is CH₃; and wherein n is a integer in the range of from 1 to 300, preferably in the range of from 1 to 200 or 2 to 150 or 2 to 100.

In a preferred embodiment, said at least one compound (c) of formula (i) is a polyalkylene glycol ether of formula (i.1)

CH₃-(CH₂)ₘ-(O-CH₂-CH₂)ₙ-OH (i.1)

or of formula (i.2)

CH₃-(CH₂)ₘ-(O-CH₂-CHCH₃)ₙ-OH (i.2)

wherein m is an integer in the range of from 5 to 25 and n is an integer in the range of from 1 to 300.

In one embodiment, m is in the range of from 11 to 23 and n is in the range of from 1 to 200 or 2 to 150 or 2 to 100.

In one embodiment, the concentration of said at least one compound (c) in the composition according to the invention is in the range of from 1 to 10 % by weight or 2 to 5 % by weight or 3 to 4 % or 3 to 3.7 % by weight based on the total weight of the composition.

In one embodiment, said at least one compound (c) is selected from the group consisting of a polyoxyethylene ether of laurylalcohol, cetyl alcohol, cetyl stearyl alcohol, stearyl alcohol, oleyl alcohol, tridecyl alcohol, or a mixture of two or more thereof.

In one embodiment, said at least one compound (c) is a polyoxyethylene stearyl ether.

In one embodiment, said polyoxyethylene stearyl ether is selected from the group consisting of polyoxyethylene (2) stearyl ether (Steareth-2), polyoxyethylene (3) stearyl ether (Steareth-3), polyoxyethylene (4) stearyl ether (Steareth-4), polyoxyethylene (5) stearyl ether (Steareth-5), polyoxyethylene (6) stearyl ether (Steareth-6), polyoxyethylene (7) stearyl ether (Steareth-7), polyoxyethylene (8) stearyl ether (Steareth-8), polyoxyethylene (10) stearyl ether (Steareth-10), polyoxyethylene (15) stearyl ether (Steareth-15), polyoxyethylene (16) stearyl ether (Steareth-16), polyoxyethylene (20) stearyl ether (Steareth-20), polyoxyethylene (21) stearyl ether (Steareth-21), polyoxyethylene (25) stearyl ether (Steareth-25), polyoxyethylene (30) stearyl ether (Steareth-30), polyoxyethylene (40) stearyl ether (Steareth-40), polyoxyethylene (50) stearyl ether (Steareth-50), polyoxyethylene (100) stearyl ether (Steareth-100) and polyoxyethylene (200) stearyl ether (Steareth-200), or a mixture of two or more thereof. The number in brackets denotes the average number of ethylene oxy-moieties contained in the compounds. E.g., polyoxyethylene (3) stearyl ether contains 3 ethylene oxy-moieties at an average.

In one embodiment, the concentration of said Steareth in the composition of the invention is from 1 % to 10 % or from 2 % to 5 % or from 3 % and 4 % or from 3 % and 3.7 % by weight based on the total weight of the composition. In one embodiment, the concentration of said Steareth in the composition of the invention is from 3 % to 3.7 % by weight and Steareth preferably is Steareth-10, Steareth-15 or Steareth-20.

### Compound (d)

The composition according to the invention further comprises at least one quaternary ammonium compound as compound (d).

Without being bound by theory, it is believed that compound (d) has the function of a surfactant in the composition according to the invention.

In a preferred embodiment, said at least one compound (d) is a compound of formula (ii):

N(R₁, R₂, R₃, R₄)⁺ X⁻ (ii)

wherein R₁, R₂, R₃, R₄ independently are acyclic C₁₋₂₄ residues, and X⁻ is selected from halogenide, methosulfate, metophosphate, phosphate, sulfate, hydroxide, carbonate and hydrogen carbonate.

In one embodiment, residues R₁, R₂, R₃, R₄ of formula (ii) independently are acyclic C₁₂₋₂₄ residues.

In another preferred embodiment, residue R₁ of the compound of formula (ii) denotes an acyclic C₁₂₋₂₄ residue, and residues R₂ to R₄ independently are C₁₋₄ alkyl residues or C₁₋₄ hydroxyalkyl residues.

The term "*acyclic residue"* encompasses both an alkyl residue and an alkene residue.

In one embodiment, the concentration of the compound of formula (ii) is from 1 to 3 % by weight or from 1.5 to 2 % or 2 % by weight based on the total weight of the composition.

In one embodiment, said at least one compound (d) of formula (ii) is selected from the group consisting of lauryl trimethyl ammonium X⁻, cetyl trimethyl ammonium X⁻, stearyl trimethyl ammonium X⁻, cetyl stearyl trimethyl ammonium X⁻, docosyl trimethyl ammonium (behentrimonium) X⁻, oleyl trimethyl ammonium X⁻; lauryl dimethyl hydroxyethyl ammonium X⁻, cetyl dimethyl hydroxyethyl ammonium X⁻, cetyl stearyl dimethyl hydroxyethyl ammonium X⁻, stearyl dimethyl hydroxyethyl ammonium X⁻, docosyl dimethyl hydroxyethyl ammonium X⁻, oleyl dimethyl hydroxyethyl ammonium X⁻.

In another preferred embodiment, said at least one compound (d) is a compound of formula (iii):

quaternary ammonium compound comprising a hydroxypropyltrimonium⁺ X⁻ moiety (iii)

wherein X⁻ is selected from halogenide, methosulfate, metophosphate, phosphate, sulfate, hydroxide, carbonate and hydrogen carbonate, preferably halogenide, methosulfate, metophosphate and phosphate.

In one embodiment, the concentration of the compound of formula (iii) is in the range of from 0.1 to 2 % by weight or from 0.25 to 0.5 % or 0.5 % by weight based on the total weight of the composition.

Compounds of formula (iii) can be derived from carbohydrates, preferably sugars or starches, or compounds containing sugar moieties or starch moieties.

Accordingly, a compound of formula (iii) encompasses a quaternary ammonium compound comprising a hydroxypropyltrimonium⁺X⁻ moiety and a carbohydrate moiety.

In one embodiment, said at least one compound (d) of formula (iii) is selected from starch hydroxypropyltrimonium X⁻, cassia hydroxypropyltrimonium X⁻ and guar hydroxypropyltrimonium X⁻; or a mixture of two or more thereof.

Compounds of formula (ii) and (iii) are known in the art or may be prepared according to known methods. Such compounds are known to function as surfactants. In one embodiment, the composition according to the invention contains as compound (d) only one or more compounds of formula (ii), i.e. no compound of formula (iii).

In a further preferred embodiment, the composition according to the invention contains as compound (d) only one or more compounds of formula (iii), i.e. no compound of formula (ii). The inventors of the present invention surprisingly have discovered that by use of a compound of formula (iii) in the composition according to the invention advantageous properties can be achieved which may solve the addressed problems, preferably confer properties with respect to long term stability.

In a further preferred embodiment, the composition according to the invention comprises both compounds (ii) and (iii). The inventors of the present invention surprisingly have discovered that by use of one or more compounds of formula (ii) in combination with one or more compounds of formula (iii) in the composition according to the invention, the advantageous properties may be further improved.

In a preferred embodiment, the compound of formula (ii) is behentrimonium X⁻, preferably behentrimonium chloride, i.e. N,N,N-trimethyldocosan-1-aminium chloride (CAS number 17301-53-0). Commercially available products are Genamin^{®} BTLF or Genamin^{®} KMDP from Clariant. In one embodiment, the concentration of behentrimonium chloride in the composition of the invention is from 1 % to 3 % or from 1.5 % and 2 % or is 2 % by weight based on the total weight of the composition.

In a further preferred embodiment, the compound of formula (ii) is lauryl dimethyl hydroxyethyl ammonium X⁻, preferably lauryl dimethyl hydroxyethyl ammonium chloride (hydroxyethyl laurdimonium chloride; CAS number 85736-63-6). A commercially available product is Praepagen^{®} HY from Clariant. In one embodiment, the concentration of hydroxyethyl laurdimonium chloride in the composition of the invention is from 1 % to 3 % or from 1.5 % and 2 % or is 2 % by weight based on the total weight of the composition.

In one embodiment, the compound of formula (iii) is a hydroxypropyltrimonium chloride, preferably starch hydroxypropyltrimonium chloride (Sensomer™ CI-50 from Lubrizol) or cassia hydroxypropyltrimonium chloride (Sensomer™ CT-250 or Sensomer™ CT-400). In one embodiment, the concentration of hydroxypropyltrimonium chloride is from 0.1 % to 2 % or from 0.25 % to 0.5% or is 0.5% by weight based on the total weight of the composition according to the invention. In one embodiment, the hydroxypropyltrimonium chloride is cassia hydroxypropyltrimonium chloride and the concentration is 0.5 % by weight based on the total weight of the composition.

In a preferred embodiment, compound (ii) is selected from behentrimonium X⁻ or from hydroxyethyl laurdimonium X- in combination with starch or cassia hydroxypropyltrimonium X⁻.

In a preferred embodiment, the composition according to the invention comprises a steareth and behentrimonium X⁻ or hydroxyethyl laurdimonium X- in combination with starch or cassia hydroxypropytrimonium X⁻, preferably wherein X- is chloride.

In one embodiment, the concentration of steareth in the composition of the invention is from 1 % to 10% by weight or from 2 % to 5 % by weight or from 3 % to 4% by weight or from 3 % to 3.7% by weight, the concentration of behentrimonium chloride or hydroxyethyl laurdimonium chloride in the composition of the invention is from 1 % to 3 % or from 1.5 % to 2.5 % or is 2 % by weight and the concentration of the starch or cassia hydrox-hydroxypropyltrimonium chloride is from 0.1 % to 2 % or from 0.25 % to 0.5 % or is 0.5% by weight based on the total weight of the composition.

In one embodiment, the steareth is Steareth-10, Steareth-15 or Steareth-20 at a concentration from 3 % to 3.7 % by weight, the behentrimonium chloride or the hydroxyethyl laurdimonium chloride is at a concentration of 1.5 to 2.5 % by weight, and the hydroxypropyltrimonium chloride is cassia hydroxypropyltrimonium chloride at a concentration of 0.2 to 0.8 % by weight. In one embodiment, the steareth is Steareth-10, Steareth-15 or Steareth-20 at a concentration of from 3 % to 3.7 % by weight, the behentrimonium chloride is at a concentration of 1.5 to 2.5 % by weight, the hydroxypropyltrimonium chloride is cassia hydroxypropyltrimonium chloride at a concentration of 0.2 to 0.8 % by weight, and the cationic polymer linked to the polymeric shell of the microcapsules is starch hydroxypropyltrimonium chloride. In one embodiment, the steareth is Steareth-10, Steareth-15 or Steareth-20 at a concentration of from 3 % to 3.7 % by weight, the behentrimonium chloride is at a concentration of 1.5 to 2.5 % by weight, and the hydroxypropyltrimonium chloride is cassia hydroxypropyltrimonium chloride at a concentration of 0.2 to 0.8 % by weight, the polymeric shell of the microcapsules is gelatin and/or carboxymethyl cellulose, and the cationic polymer linked to the polymeric shell is starch hydroxypropyltrimonium chloride.

### Compound (e)

The composition according to the invention may optionally comprise at at least one further additive as compound (e).

In one embodiment, said compound (e) is selected from the group of cationic surfactants or cationic polymers in order to further improve the stability and/or the bacteria resistance. To work efficiently as stabilizers, these products need to be cationized and the protonation can be carried out using acids known per se, for example mineral acids, mono-C₁₋₂-alkyl sulphates and/or low-molecular-weight organic carboxylic acids. A mineral acid which may be mentioned is, for example, hydrochloric acid; low-molecular-weight organic carboxylic acids which come into consideration are principally aliphatic, preferably saturated C₁₋₆-carboxylic acids, for example formic acid, acetic acid, propionic acid, lactic acid and citric acid. Of the said acids, preference is given above all to hydrochloric acid and acetic acid.

Preferred additives from the cationic surfactants family are pure or mixtures of fatty amino-esters and/or fatty amides from following the formula (III) or (IV). These products are condensation product 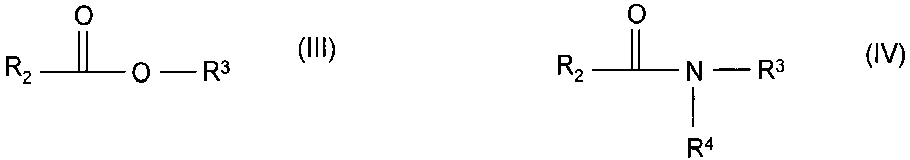 in which R₂ signifies an aliphatic hydrocarbon radical having 6 to 30 carbon atoms and especially having 7 to 24 carbon atoms, for example having 8 to 20 carbon atoms. They may be of natural or synthetic origin. The aliphatic hydrocarbon radical may also contain heteroatoms, for example oxygen, nitrogen, phosphorus and/or sulfur, but preferably not more than one heteroatom per three carbon atoms. The aliphatic hydrocarbon radicals may be linear, branched or cyclic. The hydrocarbon radicals may be saturated or unsaturated. Unsaturated hydrocarbon radicals contain one or more C=C double bonds and preferably one, two or three C=C double bonds. There is preferably no double bond in the α,β-position to the carboxyl group.

The suitable fatty aliphatic radicals may come from the corresponding fatty acids like, for example, pentanoic acid, pivalic acid, hexanoic acid, cyclohexanoic acid, heptanoic acid, octanoic acid, nonanoic acid, neononanoic acid, decanoic acid, neodecanoic acid, undecanoic acid, neoundecanoic acid, dodecanoic acid, tridecanoic acid, tetradecanoic acid, 12-methyltridecanoic acid, pentadecanoic acid, 13-methyltetradecanoic acid, 12-methyltetradecanoic acid, hexadecanoic acid, 14-methylpentadecanoic acid, heptadecanoic acid, 15-methylhexadecanoic acid, 14-methylhexadecanoic acid, octadecanoic acid, isooctadecanoic acid, eicosanoic acid, docosanoic acid and tetracosanoic acid, and also myristoleic acid, palmitoleic acid, hexadecadienoic acid, delta-9-cis-heptadecenoic acid, oleic acid, petroselic acid, vaccenic acid, linoleic acid, linolenic acid, gadoleic acid, gondoic acid, eicosadienoic acid, arachidonic acid, cetoleic acid, erucic acid, docosadienoic acid and tetra-tetracosenoic acid, and also dodecenylsuccinic acid, octadecenylsuccinic acid and mixtures thereof.

In a preferred embodiment, R³ and/or R⁴ in formulas (iii) or (IV) are each independently an alkyl radical interrupted by a heteroatom. Particularly preferred heteroatoms are oxygen and nitrogen.

For instance, R³ and R⁴ of the formula III are preferably each independently radicals

-(R⁵-O)ₙ-R⁶ (V)

in which
- R⁵: is an alkylene group having 2 to 6 carbon atoms, and preferably having 2 to 4 carbon atoms, for example ethylene, propylene, butylene or mixtures thereof,
- R⁶: is a hydrocarbon radical having 1 to 24 carbon atoms or a group of the formula -NR¹⁰R¹¹,
- n: is an integer from 2 to 500 and, preferably from 3 to 200 and especially from 4 to 50, for example from 5 to 20, and
R¹⁰, R¹¹are each independently an aliphatic radical having 1 to 24 carbon atoms and preferably 2 to 18 carbon atoms, an aryl group or heteroaryl group having 5 to 12 ring members, a poly(oxyalkylene) group having 1 to 50 poly(oxyalkylene) units, where the poly(oxyalkylene) units derive from alkylene oxide units having 2 to 6 carbon atoms or R¹⁰ and R¹¹ together with the nitrogen atom to which they are bonded form a ring having 4, 5, 6 or more ring members.

Additionally preferably, R³ and/or R⁴ are each independently radicals of the formula (VI)

-[R⁷-N(R⁸)]ₘ-(R⁹) (VI)

in which
R⁷ is an alkylene group having 2 to 6 carbon atoms and preferably having 2 to 4 carbon atoms, for example ethylene, propylene or mixtures thereof,
each R⁸ is independently hydrogen, an alkyl radical having up to 24 carbon atoms, for example 2 to 20 carbon atoms, an oxyalkylene or polyoxyalkylene radical -(R⁵-O)ₚ-R⁶, or a polyiminoalkylene radical -[R⁷-N(R⁸)]_{q}-(R⁹), where R⁵, R⁶, R⁷ and R⁸ are each as defined above and q and p are each independently 1 to 50, and
m is from 1 to 20 and preferably 2 to 10, for example three, four, five or six. The radicals of the formula VI preferably contain 1 to 50 and especially 2 to 20 nitrogen atoms.

In the case that R⁸ is hydrogen, these amines, in a specific embodiment of the process according to the invention, can also be polyamidated or imidated with the same original fatty acid.

Additives from the polycationic polymers family may comprise the following types of polymers or mixtures made of. Examples include homopolymers of the following monomers: dimethyldiallyl ammonium chloride, ethylene-imine, methacrylamido propyl trimethyl ammonium chloride, 2-methacryloyloxyethyl trimethyl ammonium chloride, 2-methacryloyloxyethyl trimethyl ammonium methylsulfate, cationic derivatives of the cellulose, such as, for example, quaternized hydroxyethylcellulose, that can acquire under denomination Polymer JR 400™ of Amerchol, cationic polymeric starches, quaternized PVP/VI such as for example Luviquat™ (BASF), products of condensation of polyglycols and amines, polymers and denominated copolymers of polyquaternium like polyquaternium-6, polyquaternium-7; polyquaternium-16, polyquaternium-10, copolymers of polyquaternium-4, , quaternized polypeptides of colagene such as, for example Lamequat^{®} (Grünau), quaternized polypeptides of wheat, cationic silicone polymers such as for example aminodimethicone or silicone quaternium-22, copolymers of acrylic acid with dimethyldiallyl ammonium chloride (Merquat^{®} 550 of Chemviron), derived cationic from Chitosan, derivatives to cationic guar gum such as for example guar-hydroxypropyltriamonnium, Jaguar^{®} CBS, Jaguar^{®} C-17, Jaguar^{®} C-16 from Celanese, quaternary polymers of ammonium salts such as, for example Mirapol^{®} A-15, Mirapol^{®} AD-1, Mirapol^{®} AZ-1 from Miranol, quaternized polysaccharide polymers of natural derivatives like agarose, selected cationic proteins among gelatin, gum arabic; cationic polymers of the group formed by polyamides, polycyanocrylates, polylactides, polyanilines, polypyroles, polyvynilpyrrolidone.

Examples of compounds (e) are triethanolamine esterquat, alkyl dimethyl benzyl ammonium chloride; coco alkyl dimethyl benzyl ammonium chloride; coco alkyl dimethyl benzyl ammonium chloride; stearidamidoethyl diethanolamine

The composition according to the invention may further comprise one or more preservatives. Preservatives are agents for protection against the damaging action of microorganisms which come into consideration are principally agents for inhibiting the growth of harmful bacteria or other microbes, or also microbicides, above all fungicides. They can be employed in very small amounts. Suitable products are generally those as are commercially available, and they can also be employed in the corresponding recommended concentrations, for example in concentrations in the range from 0.001 to 1.5 % by weight, preferably from 0.3 to 0.8 % by weight of active substance, based on the total weight of the composition according to the invention. In one embodiment, the preservative, bactericidal and/or bacteriostatic agent and/or antimicrobial and/or fungicidal agent and/or fungistatic agent is selected, for example and not restricted to, from the group formed by macrolides, pyranosides, calcium channel blockers, for example and not restricted to, cinnarizine and diltiazem; hormones, for example and not restricted to, estril, analogues thereof or thyroxine and/or its salts, caprylyl glycol, imidazolidinyl urea, methyl 4-hydroxybenzoate [INCI: methylparaben], ethyl 4-hydroxybenzoate [INCI: ethylparaben], propyl 4-hydroxybenzoate [INCI: propylparaben], butyl 4-hydroxybenzoate [INCI: butylparaben], isobutyl 4-hydroxybenzoate [INCI: isobutylparaben], 1,3-bis(hydroxymethyl)-5,5-dimethylimidazolidine-2,4-dione [INCI: DMDM Hydantoin], benzyl 4-hydroxybenzoate [INCI: benzylparaben], benzyl alcohol, dehydroacetic acid, benzoic acid, sorbic acid, salicylic acid, formic acid, propionic acid, 2-bromo-2-nitropropane-1,3-diol, 3-p-chlorophenoxy-1,2-propanodiol [INCI: chlorphenesin], dichlorobenzyl alcohol, iodopropynyl butylcarbamate, benzalkonium chloride, odor-absorbing fungicides such as zinc ricinoleate, cyclodextrins, benzethonium chloride, chlorhexidine, ethanol, propanol, 1,3-butanediol, 1,2- propylene glycol, undecylenic acid, dehydroacetic acid, *N*-methylmorpholine acetonitrile (MMA), isopropanol, methanol, 1,2-hexanediol, 1,2-octanediol, pentylene glycol, glycerin laurate, glycerin caprilate, glycerin caprate, benzoyl peroxide, chlorhexidine gluconate, triclosan and derivatives thereof, phenoxyethanol, terpinen-4-ol, α-terpineol, resorcinol, stiemycin, erythromycin, neomycin, clindamycin and its esters, tetracyclines, metronidazole, azelaic acid, tolnaftate, nystatin, clotrimazole, ketoconazole, derivatives of zinc such as zinc piritionate or trithionate, zinc oxide and zinc undecylenate, piroctone olamine, isothiazolinones, selenium sulfur, benzyl hemiformal, boric acid, sodium borate, 6,6-dibromo-4,4-dichloro-2,2'-methylenediphenol [INCI: bromochlorophene], 5-bromo-5-nitro-1,3-dioxane, tosylchloramide sodium [INCI: chloramine T], chloroacetamide, p-chloro-m-cresol, 2-benzyl-4-chlorophenol [INCI: chlorophene], dimethyl oxazolidine, dodecyl dimethyl-2-phenoxyethyl ammonium bromide [INCI: domiphen bromide], 7-ethyl bicyclooxazolidine, hexetidine, glutaraldehyde, *N*-(4-chlorophenyl)-*N*-[4-chloro-3-(trifluoromethyl)phenyl]-urea [INCI: cloflucarban], 2-hydroxy-4-isopropyl-2,4,6-cycloheptatriene-1-one [INCI: Hinokitiol], isopropylmethylphenol, mercury salts, aluminum salts, nisin, phenoxyisopropanol, o-phenylphenol, 3-heptyl-2-[(3-heptyl-4-methyl-3*H*-thiazole-2-ylidene)methyl]-4-methylthiazole iodide [INCI: Quaternium-73], silver chloride, sodium iodide, thymol, undecylenic acid, diethylenetriaminepentaacetic acid, ethylenediaminetetraacetic acid and ethylenediaminetetraacetates, lactoperoxidase, glucose oxidase, lactoferrin, alkylaryl sulfonates, halogenated phenols, phenol mercury acetate and/or mixtures thereof, benzamidines, isothiazolines, derivatives of phthalimide, derivatives of pyridine, guanidines, quinolines, 1,2-dibromo-2,4-dicyanobutane, iodine-2-propylbutyl carbamate, iodine, tamed iodines, peroxo compounds, 4-chloro-3,5-dimethylphenol, 2,2'-methylene-bis(6-bromo-4-chlorophenol), 3-methyl-4-(1-methylethyl)phenol, 3-(4-chlorophenoxy)-1,2-propanediol, 3,4,4'-trichlorocarbanilide (TTC), thiamine essence, eugenol, farnesol, glycerin monolaurate, diglycerin monocaprinate, N-alkyl salicylic acid amides such as *n*-octyl salicylic acid amide or *n*-decyl salicyl-salicylic acid amide, derivatives of halogenated xylene and cresol, such as p-chloro-meta-cresol or p-chloro-meta-xylene, extracts of *Allium sativum, Calendula officinalis, Chamomilla recutita*, *Echinacea Purpura, Hyssopus Officinalis, Melaleuca alternifolia* or tea tree oil, carnation essence, menthol and mint essence, among others.

In a ***second aspect,*** this invention relates to a process for the manufacture of the composition according to the invention by thermal treatment of a mixture comprising:
(a) at least one cationic microcapsule having a shell and a core, wherein the microcapsule comprises at least one active agent;
(b) water;
(c) at least one non-ionic oxygen-containing compound comprising at least one alkylene group;
(d) at least one quaternary ammonium compound.

The composition according to the invention, preferably in the form of an aqueous dispersion, can be produced by mixing at least one microcapsule (a) with water (b) and the further compounds (c) and (d) and optionally (e). The mixing of the components can take place at any desired suitable temperatures, for example in the range from 15 °C to the reflux temperature, or in the range of from 18 to 90 °C or or temperatures of >50 °C.

The composition according to the invention, preferably in the form of defined aqueous compositions, can be transported, stored and handled in the same form as they have been produced. They are suitable as finishing agents and can be employed directly, in the same form as they have been formulated, for example to a dry substance content of from 2 to 10 % by weight.

The instant aqueous compositions of cationically charged microcapsules prove to have a good stability, particularly storage and transport stability, and can rapidly be diluted to any desired extent with water. The aqueous compositions of cationically charged microcapsules according to the invention, both in dissolved form and in emulsified form, in concentrated or also dilute form, and the liquors produced therefrom possess an excellent shear force stability, and are, in particular, stable and have an unchanged action even under strong dynamic loading of liquor and/or textile material. Furthermore, the composition of cationically charged microcapsules of the invention is suitable for impregnation processes in which high shear forces act, for example for spraying-on, if desired in the form of foam (in which the composition is forced through spray nozzles in order to spray it onto the goods) or for vacuum impregnation processes (in which the goods sprayed with the treatment composition are fed over at least one vacuum sieve so that the excess of composition is removed vigorously and rapidly by suction and fed back to the impregnation using a circulation pump).

Therefore, a ***third aspect*** of this invention relates to a substrate containing the composition of cationically charged microcapsules of the invention. Suitable substrates that can be treated with the composition of the invention are any desired fibrous material or textile material, as occurs in the textile industry, namely natural or also synthetic or semisynthetic materials and blends thereof, for example material containing natural, modified or regenerated cellulose [principally cotton ("CO") or viscose], natural or synthetic polyamide [principally wool ("WO") or fully synthetic polyamides ("PA")], polyester ("PES"), polyurethane ("PU") or polyacrylonitrile ("PAN"), and blends thereof (for example PES/CO, PES/WO, PA/PU and PAN/CO), for example also elastane. The material can be in any desired processing form, for example as loose fibres, filaments, threads, yarn strands and bobbins, woven fabrics, knitted fabrics (for example tricot), bonded or non-bonded nonwovens, felts, carpets, velvet, terry cloth or tufted fabrics or also ready-made or half-ready-made goods. The finish is preferably applied to cross-wound bobbins ("cheeses"), textile webs, textile tubular goods (for example knitted tubular goods) or piece goods. Examples of finished goods are bandages, gauzes, t-shirts, socks, tights, underwear, girdles, gloves, diapers, sanitary napkins, dressings, bedspreads, wipes, adhesive patches, non-adhesive patches, micro-electric patches and/or face masks.

The finishing is advantageously carried out from aqueous, distinctly acidic to nearly neutral medium, for example in the pH range 4.0 - 8.5. The concentration of the compositions of the invention, based on the substrate, can vary in broad limits depending on the type and nature of the substrate and the desired effect and is - calculated on component (a) - advantageously at values in the range from 0.02 % to 30 %, from 0.05 % to 15 % by weight of cationically charged microcapsules, based on the dry weight of the substrate.

The finishing process of the invention is advantageously carried out as the final finishing step of the material, preferably after bleaching, an optical brightening process and/or a dyeing process, if desired together with an additional treatment, for example permanent finishing (synthetic resin finishing) of the fiber material. The finishing can be carried out by any desired processes which are conventional per se, for example by impregnation methods or by exhaust methods. In exhaust methods, methods from long liquor and also from short liquor are suitable, for example at liquor goods ratios in the range from 100:1 to 0.5:1, from 60:1 to 2:1; the application temperature can also be at the usual values, for example in the range between room temperature and 60 °C, in the range from 25 °C to 50 °C, and the pH is in the range from 5 to 8.5. The impregnation can likewise be carried out by methods which are conventional per se, for example by dipping, padding, foam application or spraying, preferably at temperatures of 15 - 40 °C and at pH values in the range from 4 to 8. After impregnation or after application by an exhaust method, the treated goods can be dried in a conventional manner, for example at from 30 to 190 °C, or from 60 to 170 °C.

A ***fourth aspect*** of this invention relates to a fabric softener containing the composition of cationically charged microcapsules of the invention. The composition of cationically charged microcapsules is mixed with the further components of the fabric softener at the desired concentration and at any temperature suitable for the chemical or physical stability of the components of the fabric softener.

A ***fifth aspect*** of this invention relates to the use of the substrate containing the composition of cationically charged microcapsules of the invention for the cosmetic, non-therapeutic treatment and/or care of the skin, hair and/or scalp. In one embodiment, the cosmetic, non-therapeutic treatment and/or care of the skin, hair and/or scalp is selected from the group formed by the treatment and/or prevention of skin aging, firming of the skin, whitening of the skin and/or moisturizing of the skin.

Accordingly, the active agent is selected from cosmetic active agents.

When the term *"treatment'* is accompanied by the qualifications *"cosmetic, non-therapeutic"* they refer to the application of the compound to the skin and/or mucous membranes in particular with the aim of improving the cosmetic qualities of the skin and/or mucous membranes such as and not restricted to, their level of hydration, elasticity, firmness, shine, tone or texture, among others. The term *"care"* in this invention refers to the maintenance of the qualities of the skin and/or mucous membranes. These qualities are subject to improvement and maintained through a cosmetic treatment and/or care of the skin and/or mucous membranes both in healthy subjects as well as those which present diseases and/or disorders of the skin and/or mucous membranes, such as and not restricted to, ulcers and lesions on the skin, psoriasis, dermatitis, acne or rosacea, among others.

If the cationic microcapsules used in the composition according to the invention comprise active agents, which have pharmaceutical activity, a substrate comprising said composition may be used in a therapeutic application.

Accordingly, a sixth ***aspect*** of this invention relates to a substrate comprising the composition according to the invention for use as a medicament.

In one embodiment, the invention relates to a substrate comprising the composition according to the invention for use in the treatment of the skin. In one embodiment, the treatment of the skin is healing of the skin and/or dermatological treatment of skin diseases.

The term *"treatment",* as used in the context of this specification when it is not accompanied by the qualifications *"cosmetic, non-therapeutic",* means the administration of a compound according to the invention to alleviate or eliminate a disease or disorder or reduce or eliminate one or more symptoms associated with this disease or disorder. The term *"treatment"* also covers the ability to alleviate or eliminate the physiological consequences of the disease or disorder.

### EXAMPLES

Each of the documents referred to above is incorporated herein by reference, including any prior applications, whether or not specifically listed above, from which priority is claimed. The mention of any document is not an admission that such document qualifies as prior art or constitutes the general knowledge of the skilled person in any jurisdiction. Except in the Examples, or where otherwise explicitly indicated, all numerical quantities in this description specifying amounts of materials, reaction conditions, molecular weights, number of carbon atoms, and the like, are to be understood as modified by the word "about." It is to be understood that the upper and lower amount, range, and ratio limits set forth herein may be independently combined. Similarly, the ranges and amounts for each element of the invention can be used together with ranges or amounts for any of the other elements.

While certain representative embodiments and details have been shown for the purpose of illustrating the subject invention, it will be apparent to those skilled in this art that various changes and modifications can be made therein without departing from the scope of the subject invention. In this regard, the scope of the invention is to be limited only by the following claims.

### General Methodology

All the reagents and solvents are of synthesis quality and were used without any additional treatment.

### Example 1: Preparation of cationic microcapsules

Microcapsules were prepared using the ingredients listed below:

| INGREDIENT (INCI Nomenclature) | | % IN WEIGHT |
|---|---|---|
| A | AQUA (WATER) | 28.6 |
| A | PROPANEDIOL | 2.7 |
| A | PHENOXYETHANOL | 0.54 |
| B | GELATIN | 1.5 |
| B | CELLULOSE GUM | 0.38 |
| C1 | AQUA (WATER) | 5.76 |
| C1 | GLYCERIN | 1.74 |
| C1 | LECITHIN | 0.92 |
| C1 | CAFFEIN | 0.63 |
| C1 | BUTCHERBROOM (RUSCUS ACULEATUS) ROOT EXTRACT, MALTODEXTRIN, SILICA [Commercial Butcherbroom extract] | 0.61 |
| C1 | BUTYLENE GLYCOL, WATER (AQUA), IVY (HEDERA HELIX) EXTRACT [Commercial Hedera Helyx extract] | 0.41 |
| C1 | CARNITINE | 0.32 |
| C1 | CAPRYLYL GLYCOL | 0.097 |
| C1 | PHENOXYETHANOL | 0.097 |
| C1 | ESCIN | 0.079 |
| C1 | XANTHAN GUM | 0.043 |
| C1 | POTASSIUM SORBATE | 0.032 |
| C1 | CARRAGEENAN (CHONDRUS CRISPUS) | 0.0054 |
| C1 | TRIPEPTIDE-1 | 0.00107 |
| C2 | SOYBEAN (GLYCINE SOJA) OIL | 7.1 |
| C2 | TOCOPHERYL ACETATE | 1.0 |
| C2 | CETYL PEG/PPG-10/1 DIMETHICONE | 0.96 |
| C2 | SORBITAN TRISTEARATE | 0.38 |
| C2 | ACETYLARGINYLTRYPTOPHYL DIPHENYLGLYCINE [RELISTASE™] | 0.0015 |
| D | CITRIC ACID | 0.096 |
| D | AQUA (WATER) | 0.6 |
| E | SODIUM HYDROXIDE | 0.05 |
| E | AQUA (WATER) | 0.2 |
| F | GLUTARAL | 0.23 |
| F | AQUA (WATER) | 6.6 |
| G | AQUA (WATER) | 0.8 |
| G | SODIUM METABISULFITE | 0.21 |
| H | WATER (AQUA), STARCH HYDROXYPROPYLTRIMONIUM CHLORIDE, UREA, SODIUM LACTATE, SODIUM CHLORIDE, SODIUM BENZOATE [SENSOMER™ CI-50] | 11.3 |
| I | SCLEROTIUM GUM | 1.0 |
| J | AQUA (WATER) | q.s.p. |

Preservatives (PHENOXYETHANOL and PROPANEDIOL) were mixed with water to obtain A. The polymers which formed the shell of the microcapsules (Phase B) were added and the resulting mixture (A+B) was heated up to 65 °C until homogeneity.

The mixture C, which contained the active ingredients, was prepared following this procedure: First, C2 was melt at 75 °C until total dispersion of the peptide; the component C1 (LIPOREDUCTYL^{®} CT), previously preheated to 65 °C, was added to C2 with intense stirring to obtain an O/W emulsion.

The emulsion formed by C1 and C2 was added to A+B, and the temperature was maintained at 65 °C. Then, pH was adjusted with citric acid (component D) to 4.4, and stirring was maintained for 30 min. From this point, the temperature was no longer maintained at 65 °C. pH was raised to 6.5 with sodium hydroxide (component E) and stirring was maintained for 30 min.

The cross-linker glutaraldehyde (component F, GLUTARAL) was added and after 1 h of stirring, the pH was adjusted to 4.6 with 0.5% of a 40% citric acid solution. Then sodium metabisulfite (component G) was added, and after 1 h of stirring the cationic polymer component H (Starch Hydroxypropyltrimonium Chloride Urea) was added. Finally, pH was adjusted to 3.9 with 1 % of a 40% citric acid solution.

A mixture of the thickener component I and J was obtained by mixing the components by stirring and maintaining the stirring for 2 h. This mixture of I+J was added to the previous mixture and stirring was maintained for 30 additional minutes.

### Example 2: Preparation of cationic microcapsules

The preparation process was identical to Example 1, with the following list of ingredients:

| INGREDIENT (INCI Nomenclature) | | % IN WEIGHT |
|---|---|---|
| A | AQUA (WATER) | 28.9 |
| A | PROPANEDIOL | 2.7 |
| A | PHENOXYETHANOL | 0.54 |
| B | GELATIN | 1.5 |
| B | CELLULOSE GUM | 0.38 |
| C1 | PSEUDOALTEROMONAS FERMENT EXTRACT [ANTARCTICINE^{®}] | 3.6 |
| C1 | WATER (AQUA), GLYCERIN, PSEUDOALTEROMONAS FERMENT EXTRACT, XANTHAN GUM, PROLINE, ALANINE, SERINE, SODIUM PHOSPHATE, SODIUM HYDROXYDE, ETHYLHEXYLGLYCERIN, CAPRYLYL GLYCOL [XPERTMOIST^{®}] | 7.5 |
| C2 | SOYBEAN (GLYCINE SOJA) OIL | 6.5 |
| C2 | TOCOPHERYL ACETATE | 1.0 |
| C2 | LECITHIN | 0.9 |
| C2 | CETYL PEG/PPG-10/1 DIMETHICONE | 0.32 |
| C2 | SORBITAN TRISTEARATE | 0.13 |
| D | CITRIC ACID | 0.096 |
| D | AQUA (WATER) | 0.64 |
| E | SODIUM HYDROXIDE | 0.05 |
| E | AQUA (WATER) | 0.2 |
| F | GLUTARAL | 0.23 |
| F | AQUA (WATER) | 6.5 |
| G | AQUA (WATER) | 0.75 |
| G | SODIUM METABISULFITE | 0.21 |
| H | WATER (AQUA), STARCH HYDROXYPROPYLTRIMONIUM CHLORIDE, UREA, SODIUM LACTATE, SODIUM CHLORIDE, SODIUM BENZOATE [SENSOMER™ CI-50] | 11.3 |
| I | SCLEROTIUM GUM | 1.0 |
| J | AQUA (WATER) | q.s.p. |

The cationic microcapules obtained in Examples 1 and 2 were dispered in water using different dispersants (c) and/or surfactants (d). In order to be applicable for use, the dispersion needs to be:
- fluid enough (viscosity < 200 mPa·s);
- homogeneous, avoiding decantation of microcapsules during the application into fabric;
- stable over storage time.

Aqueous dispersions were prepared, all of them including 13.3 % [ceggr1] by weight of microcapsules and 0.1 % by weight of odour masker UNISTAB S-69 (FARNESOL, LINALOOL, Induchem AG) based on the total amount of the aqueous dispersion. If desired, a biocide was added (NEOLONE™ 950, Methylisothiazoline, Rohm & Haas).

The compounds according to compounds (c) and (d) used in the following examples were the following ones listed in Table 1:

**Table 1**

| Ingredient | Supplier | INCI NAME |
|---|---|---|
| *Compound (c), formula (i)* | | |
| ALK-ST-10 | Clariant AG | STEARETH-10 |
| ALK-ST-15 | Clariant AG | STEARETH-15 |
| ALK-ST-20 | Clariant AG | STEARETH-20 |

| *Compound (d), formula (ii)* | | |
|---|---|---|
| GENAMIN BTLF | Clariant AG | BEHENTRIMONIUM CHLORIDE DIPROPYLENE GLYCOL |
| GENAMINE KMDP | Clariant AG | Behenyl trimethyl ammonium chloride; |
| GENAMIN CTAC | Clariant AG | Alkyl hydroxyethyl dimethyl ammonium chloride |
| PRAEPAGEN HY | Clariant AG | Cetyl trimethyl ammonium chloride |
| PRAEPAGEN WB | Clariant AG | Distearyl dimethyl ammonium chloride. |

| *Compound (d), formula (iii)* | | |
|---|---|---|
| SENSOMER CT-400 | LUBRIZOL ADVANCED MATERIALS | CASSIA HYDROXYPROPYLTRIMONIU M CHLORIDE WATER (AQUA) |

| SENSOMER CT-250 | LUBRIZOL ADVANCED MATERIALS | Cassia hydroxypropyltrimonium chloride |
|---|---|---|
| *Compound (e)* | | |
| PRAEPAGEN TQ | Clariant AG | Triethanolamine esterquat |
| DODIGEN 1611 | Clariant AG | Alkyl dimethyl benzyl ammonium chloride |
| DODIGEN 226 | Clariant AG | Coco alkyl dimethyl benzyl ammonium chloride |
| DODIGEN 800 | Clariant AG | Coco alkyl dimethyl benzyl ammonium chloride |
| CERANINE HC | | Stearidamidoethyl diethanolamine |

### Example 3 (Comparative)

A 500 millilitre reactor (glass) was equipped with an anchor stirrer, a thermometer, a dropping funnel and a reflux condenser. To this reactor, 300 millilitres of water [compound (b)] were introduced under stirring. The water was heated to 80 °C and 39 grams of cationic microcapsules prepared according to Example 1 or 2 [component/compound (a)] were introduced via the dropping funnel by vacuum. This mixture was stirred for 5 minutes. Stirring was then stopped and the calculated amount of compound or compounds according to (c) or (d) was added, if necessary previously heated to 80 °C. The reaction vessel was kept under stirring at 80 °C for 3 hours. The reaction mixture was cooled to room temperature, and the biocide was added, if desired. The mixture was then filtered on a GAF 100 µm. Appearance: white to light beige dispersion.

The stability was checked according the following procedure: Two samples were kept at room temperature and at 40 °C, and were analysed and compared to the original results after 1 week, 2 weeks, 1 month, 2 months and 3 months. An additional sample kept at 60 °C was analyzed after 1 and 2 weeks. One or more of the parameters analysed were the following: colour, odour, physical appearance pH and viscosity. Viscosity was determined using a rotational viscosimeter at 23 °C.

The results are listed in Table 2:

**Table 2**

| Ex. | Compound (c) or (d) | Decantation | Viscosity |
|---|---|---|---|
| 3a | 3 % ALK-ST-10 | Unstable after 2 weeks storage time. Not acceptable | < 200 mPas |
| 3b | 3 % ALK-ST-15 | Unstable after 1 week storage time. Not acceptable | < 200 mPas |
| 3c | 3 % ALK-ST-20 | Unstable after 1 week storage time. Not acceptable | < 200 mPas |
| 3d | 3 % GENAMIN BTLF | Unstable after 1 week storage time | < 200 mPas |

The compositions according to Examples 3a to 3d were not acceptable for use in textile application.

**Example 4: Preparation of a composition according to the invention**

Example 3 was repeated with the difference that 4 g ALK-ST-10 [compound (c)], 4 g GENAMIN BTLF [compound (d), formula (ii)] and 0.5 g SENSOMER CT-400 [compound (d), formula (iii)] were introduced into the vessel under stirring.

The results are listed in Table 3:

**Table 3**

| Ex. | Stabilizing additive/s | Preservatives (additional to the ones contained in cationic microcapsules) | Microbial safety (microbial stress test) | Stability / Viscosity |
|---|---|---|---|---|
| 4 | 3.7% ALK-ST-10 2% GENAMIN BTLF 0.5% SENSOMER CT-400 | 0.09 % NEOLONE 950 | acceptable | Stable; < 200 mPas Acceptable |

**Example 5: Preparation of a composition according to the invention**

Example 3 was repeated with the compounds listed in Table 4.

The stability of each example was checked according to the following procedure: Two samples were kept at room temperature and at 50 °C, and were analysed and compared to the original results after 1 month.

The results are listed in Table 4:

**Table 4**

| Ex. | Stabilizing Additive/s | Stability, appearance | Textile application | Viscosity |
|---|---|---|---|---|
| 5a | 4 % ALK-ST-10 | acceptable | acceptable | < 200 mPas |
| | 1 % PRAEPAGEN HY | | | |
| | 0.5% SENSOMER CT-400 | | | |
| | 1.5 % GENAMIN CTAC | | | |
| 5b | 3.7 % ALK-ST-10 | acceptable | acceptable | < 200 mPas |
| | 2 % GENAMINE BTLF | | | |
| | 0.5% SENSOMER CT-400 | | | |
| 5c | 3.0 % ALK-ST-15 | acceptable | acceptable | < 200 mPas |
| | 2 % GENAMINE BTLF | | | |
| | 0.5% SENSOMER CT-400 | | | |
| 5d | 3 % ALK-ST-10 | acceptable | acceptable | < 200 mPas |
| | 1.5 % DODIGEN 1611 | | | |
| | 0.5% SENSOMER CT-400 | | | |
| 5e | 3.7 % ALK-ST-10 | acceptable | acceptable | < 200 mPas |
| | 2 % GENAMINE KMDP | | | |
| | 0.5% SENSOMER CT-400 | | | |
| 5f | 4 % ALK-ST-10 | acceptable | acceptable | < 200 mPas |
| | 1 % PRAEPAGEN HY | | | |
| | 0.5% SENSOMER CT-250 | | | |
| | 1.5 % GENAMIN CTAC | | | |
| 5g | 3.7 % ALK-ST-10 | acceptable | acceptable | < 200 mPas |
| | 2 % GENAMINE BTLF | | | |
| | 0.5% SENSOM ER CT-250 | | | |
| 5h | 3.0 % ALK-ST-15 | acceptable | acceptable | < 200 mPas |
| | 2 % GENAMINE BTLF | | | |
| | 0.5% SENSOMER CT-250 | | | |
| 5i | 3 % ALK-ST-10 | acceptable | acceptable | < 200 mPas |
| | 1.5 % CERANINE HC | | | |
| | 0.5% SENSOMER CT-250 | | | |
| 5j | 3.7 % ALK-ST-10 | acceptable | acceptable | < 200 mPas |
| | 2 % GENAMINE KMDP | | | |
| | 0.5% SENSOMER CT-250 | | | |

## Claims

1. Composition comprising:
(a) at least one cationic microcapsule having a shell and a core, wherein the microcapsule comprises at least one active agent;
(b) water;
(c) at least one non-ionic oxygen-containing compound comprising at least one alkylene group;
(d) at least one quaternary ammonium compound.

2. Composition of claim 1, wherein the shell of the microcapsule consists or comprises at least a cationic polymer and/or is partially covered with at least a cationic compound.

3. Composition of claim 1 or 2, wherein the active agent is selected from the group consisting of cosmetic and/or pharmaceutical active agents and insect repellants.

4. Composition of any one of the preceding claims, wherein said at least one compound (c) is a polyalkylene glycol ether of formula (i)
R₁-O-(CH₂-CHR₂)ₙ-OH (i)
wherein R₁ is a branched or unbranched alkyl or alkenyl group having from 6 to 26 carbon atoms; and R₂ is H or CH₃; or said ether of formula (i) comprises moieties in which R₂ is H and moieties in which R₂ is CH₃; and wherein n is a integer in the range of from 1 to 300.

5. Composition of any one of the preceding claims, wherein said at least one compound (c) is selected from the group consisting of a polyoxyethylene ether of laurylalcohol, cetyl alcohol, cetyl stearyl alcohol, stearyl alcohol, oleyl alcohol, and tridecyl alcohol, and a mixture of two or more thereof.

6. Composition of any one of the preceding claims, wherein the concentration of said at least one compound (c) is in the range of from 1 to 10 % by weight or 2 to 5 % by weight or 3 to 4 % by weight based on the total weight of the composition.

7. Composition of any one of the preceding claims, wherein said at least one compound (d) is selected from the group consisting of a compound of formula (ii)
N(R₁, R₂, R₃, R₄)⁺ X⁻ (ii)
wherein R₁, R₂, R₃, R₄ independently are acyclic C₁₋₂₄ residues;
or wherein
R₁ is an acyclic C₁₂₋₂₄ residue, and R₂ to R₄ independently are C₁₋₄ alkyl residues or C₁₋₄ hydroxyalkyl residues; and
X⁻ is selected from halogenide, methosulfate, metophosphate, phosphate, sulfate, hydroxide, carbonate and hydrogen carbonate, preferably halogenide, methosulfate, metophosphate and phosphate;
and a compound of formula (iii)
quaternary ammonium compound comprising a hydroxypropyltrimonium⁺
X- moiety (iii)
wherein X⁻ is selected from halogenide, methosulfate, metophosphate, phosphate, sulfate, hydroxide, carbonate and hydrogen carbonate, preferably halogenide, methosulfate, metophosphate and phosphate;
and a mixture of compounds (ii) and (iii).

8. Composition of claim 7, wherein said compound of formula (iii) is a quaternary ammonium compound comprising a hydroxypropyltrimonium⁺X⁻ moiety and a carbohydrate moiety.

9. Composition of any one of claims 7 to 8, wherein the composition comprises at least one compound of formula (iii) or comprises at least one compound of formula (ii) and at least one compound of formula (iii).

10. Composition of any one of claims 7 to 9, wherein the concentration of the compound of formula (ii) is from 1 to 3 % by weight or from 1.5 to 2 % by weight based on the total amount of the composition and/or wherein the concentration of the compound of formula (iii) is in the range of from 0.1 to 2 % by weight or from 0.25 to 0.5 % by weight based on the total amount of the composition.

11. Substrate comprising the composition according to any one of claims 1 to 10.

12. Fabric softener comprising the composition according to any one of claims 1 to 10.

13. Use of the substrate of claim 11 for the cosmetic, non-therapeutic treatment and/or care of the skin, hair and/or scalp.

14. Substrate of claim 11 for use as a medicament.

15. Substrate of claim 11 for use in the treatment of the skin and/or scalp and/or in the prevention of skin diseases.
